# EUROPEAN PATENT APPLICATION

(11) **EP 4 488 267 A1**
(43) Date of publication of application: **08.01.2025**
(21) Application number: 23763047.0
(22) Date of filing: 02.03.2023
(51) Int. Cl.: C07D 401/02, C07D 403/02, A61K 31/435, A61P 25/00

(54) **HISTAMINE H3 RECEPTOR ANTAGONIST AND MEDICAL USE THEREOF**

(30) Priority: 02.03.2022 CN 202210200094
(71) Applicant: Hangzhou Bio-Sincerity Pharma-tech Co., Ltd., Hangzhou, Zhejiang 311100 (CN)
(72) Inventor: FENG, Enguang, Hangzhou, Zhejiang 311100 (CN); QIN, Jinjing, Hangzhou, Zhejiang 311100 (CN); JIN, Lifeng, Hangzhou, Zhejiang 311100 (CN); JIN, Zewu, Hangzhou, Zhejiang 311100 (CN); LI, Yuanyuan, Hangzhou, Zhejiang 311100 (CN); XIONG, Xiaohong, Hangzhou, Zhejiang 311100 (CN); LEI, Shaowei, Hangzhou, Zhejiang 311100 (CN); SHEN, Ximing, Hangzhou, Zhejiang 311100 (CN); LOU, Jinfang, Hangzhou, Zhejiang 311100 (CN)
(74) Representative: Huang, Liwei
(86) International application number: PCT/IB2023/000170
(87) International publication number: WO 2023/166351

(57) **Abstract**

The present invention discloses a structurally novel histamine H3 receptor antagonist, specifically relates to the compound of free base, or isomer, or solvate, or pharmaceutically acceptable salt thereof; methods of preparing compound; compound composition, and therapeutic uses thereof. The present invention has developed a series of structurally novel compounds based on histamine H3 receptor ligands, and a series of relevant biological tests have been performed on the compounds. The results of the tests all indicate that the compounds have significant H3 receptor antagonistic activity, and can be used as a lead compound for the prevention or treatment H3 receptor-related diseases.

## Description

### FIELD OF THE INVENTION

The invention belongs to the field of pharmaceutical technology and relates to a histamine H3 receptor antagonist and pharmaceutical use thereof, and specifically relates to the compound of free base, or isomer, or solvate, or pharmaceutically acceptable salt thereof, preparation methods of the compound, composition, and therapeutic uses thereof of the compounds.

### BACKGROUND OF THE INVENTION

Following the Human Genome Project, the histamine receptor family has expanded to four distinct G protein-coupled receptors (GPCRs): the H1, H2, H3, and H4 receptors (Nature Review Drug Discovery, 2005, 4, 107-120). Histamine H3 receptors are mainly expressed in the central nervous system and less frequently in the peripheral nervous system.

Histamine H3 receptor (H3R) is widely expressed in the brain, mainly in the cerebral cortex, hippocampus, amygdala, striatum and other areas closely related to memory and cognitive abilities. Blocking these receptors with selective antagonists/inverse agonists can increase the release of neurotransmitters such as acetylcholine, dopamine or 5-hydroxytryptamine, thereby regulating a variety of neuropathic behaviors such as learning and memory, wakefulness and sleep (British Journal of Pharmacology, 2008, 154(6), 1166-1181).

Literatures suggests that ligands of histamine H3 receptor can be used to treat cognitive impairment (British Journal of Pharmacology, 2008, 154(6), 1166-1181), dementia (Drug News Perspective, 2010, 23(2), 99-103), attention deficit hyperactivity disorder, obesity (Indian Journal of Pharmacology, 2001, (33), 17-28), schizophrenia (Biochemical Pharmacology, 2007, 73(8), 1215-1224), and pain (Journal of Pharmacology and Experimental Therapeutics, 2011, 336(1), 30-37).

Presently, histamine H3 receptor ligands compounds are mainly divided into two categories: imidazole ring substituted at the 4 (5) position and non-imidazole ring. Compounds containing imidazole rings have the disadvantages of low blood-brain barrier penetration, interaction with cytochrome P-450 proteins, liver toxicity, and ocular toxicity. Representative patents of non-imidazole ring compounds: ①US20020177589A1 discloses new ligands that can be used to regulate histamine H3 receptor. The compounds in this patent can be used to treat Alzheimer's disease and cognitive impairment. The representative compound is ABT-239, which has serious cardiac side effects in clinical trials, resulting in the termination of clinical trials. ② WO2012114348A1 discloses novel compounds as ligands of histamine H3 receptor, which can be used to treat various diseases, such as cognitive impairment, dementia, etc. Samelisant (SUVN-G3031), an H3R antagonist/inverse agonist for the treatment of cognitive impairment, is currently under development. The drug's Phase II clinical indication is narcolepsy, and the Phase I clinical trial for cognitive impairment was conducted from 2015 to 2017, with no subsequent progress. ③WO2014030170A1 discloses pyridazinone derivatives as histamine H3 inhibitor, as well as their uses, preparation methods and pharmaceutical compositions. The inventive compounds can be used to treat various diseases such as cognitive impairment, sleep/wakefulness disorders, eating disorders, etc., but the clinical trial was terminated due to the unsatisfactory PK/PD data.

In summary, while a number of histamine H3 receptor ligands analogs are disclosed, only Pitolisant (H3R antagonist/inverse agonist) has been approved for marketing in this area of research and development to date, with the marketed indication of narcolepsy. SUVN-G3031 and other investigational drugs in Phase 2 clinical studies are still narcolepsy, the clinical studies in neurological disorders such as cognitive impairment have been declared failures.

Therefore, there is an urgent need to discover a new compound with novel structure, high safety profile, and better drug's efficacy than Pitolisant and SUVN-G3031, which is used to prevent or treat a disease associated with H3 targets. This is still not solved by those skilled in the art.

### SUMMARY OF THE INVENTION

The present invention aims to provide a structurally novel histamine H3 receptor antagonist which can be used to prevent or treat the diseases associated with histamine H3 receptor.

To achieve the above-mentioned purpose of the invention, the technical solution provided by the present invention is as follows:
A histamine H3 receptor antagonist, which is a compound of general formula I or a pharmaceutically acceptable salt thereof: 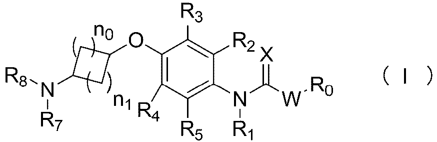
wherein:
   W is selected from -(CH₂)ₘ or -NR₆(CH₂)ₘ-;
   X is selected from O or S;
   n₀ and n₁ are each independently selected from 1, 2 or 3; m is selected from 0, 1, 2, 3, 4, 5 or 6;
   R₀ is selected from -NR'R", -O(CH₂)ₘNR'R", substituted or unsubstituted 5-8 membered heterocyclyl, substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl or heteroaryl;
   R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
   R₁ and R₆ are each independently selected from hydrogen, C₁₋₆ alkyl, -C(O)-alkyl or -S(O)₂-alkyl;
   R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
   or, R₂ and R₃ together with the carbon atoms on the benzene ring to which they are attached form a substituted or unsubstituted benzobicyclic structure, wherein the benzobicyclic structure can be but not limited to, a benzoheterocyclic ring;
   or, R₄ and R₅ together with the carbon atoms on the benzene ring to which they are attached form a substituted or unsubstituted benzobicyclic structure, wherein the benzobicyclic structure can be but not limited to, a benzoheterocyclic ring;
   R₇ and R₈ are each independently one or more selected from hydrogen, halogen, C₁₋₆ alkyl, C₂₋₆ alkenyl, C₂₋₆ alkynyl, amino, cyano, amide, sulfone, sulfoxide, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, C₁₋₆ alkoxyamide, C₁₋₆ alkoxysulfone and C₁₋₆ alkoxysulfoxide;
   or, R₇ and R₈ together with the nitrogen atom to which they are attached form a ring, and the substituents on the ring are selected from one or more of the following groups: hydrogen, halogen, C₁₋₆ alkyl, C₁₋₆ alkoxy, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-8 membered heterocyclyl, and 3-8 membered heteroaryl;
   the heterocyclyl, heteroaryl and benzoheterocyclic group contain at least one heteroatom, and the heteroatom is selected from N, O or S.

Preferably, the compound of general formula II or a pharmaceutically acceptable salt thereof: 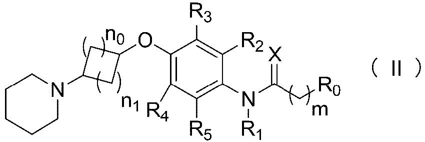 wherein:
X is selected from O or S;
n₀ and n₁ are each independently selected from 1, 2 or 3; m is selected from 0, 1, 2, 3, 4, 5 or 6;
R₀ is selected from -NR'R", -O(CH₂)ₘNR'R", substituted or unsubstituted 5-8 membered heterocyclyl, and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₁ is selected from hydrogen, C₁₋₆ alkyl, -C(O)-alkyl or -S(O)₂-alkyl;
R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
or, R₂ and R₃ together with the carbon atoms on the benzene ring to which they are attached form a substituted or unsubstituted benzobicyclic structure, wherein the benzobicyclic structure can be but not limited to, a benzoheterocyclic ring;
or, R₄ and R₅ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

Preferably, the compound of general formula II-1 or a pharmaceutically acceptable salt thereof: 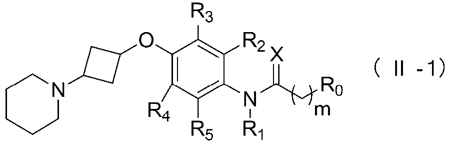 wherein:
X is selected from O or S;
m is selected from 1 or 2;
R₀ is selected from substituted or unsubstituted 5-8 membered heterocyclyl and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R₁ is selected from hydrogen, C₁₋₆ alkyl, -C(O)-alkyl or -S(O)₂-alkyl;
R₂, R₃, R₄, and R₅ are each independently one or more selected from hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
the heterocyclyl and heteroaryl contain at least one heteroatom, and the heteroatom is selected from N, OorS.

Preferably, the compound of general formula II-2 or a pharmaceutically acceptable salt thereof: 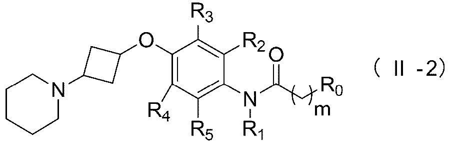 wherein:
m is selected from 1 or 2;
R₀ is selected from -NR'R" or -O(CH₂)mNR'R";
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₁ is selected from hydrogen, C₁₋₆ alkyl, -C(O)-alkyl or -S(O)₂-alkyl;
R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide.

Preferably, the compound of general formula III or a pharmaceutically acceptable salt thereof: 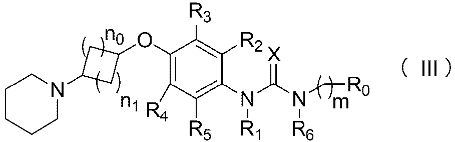 wherein:
X is selected from Oor S;
n₀ and n₁ are each independently selected from 1, 2 or 3; m is selected from 0, 1, 2, 3, 4, 5 or 6;
R₀ is selected from substituted or unsubstituted 5-8 membered heterocyclyl and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R₁ and R₆ are each independently selected from hydrogen, C₁₋₆ alkyl, -C(O)-alkyl or -S(O)₂-alkyl;
R₂, R₃, R₄, R₅ and R₆ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
or, R₂ and R₃ together with the carbon atoms on the benzene ring to which they are attached form a substituted or unsubstituted benzobicyclic structure, wherein the benzobicyclic structure can be but not limited to, a benzoheterocyclic ring;
or, R₄ and R₅ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

Preferably, the compound of general formula III-1 or a pharmaceutically acceptable salt thereof: 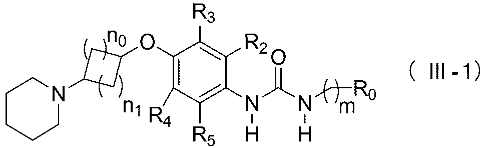 wherein:
n₀ and n₁ are each independently selected from 1, 2 or 3; m is selected from 0, 1, 2, 3, 4, 5 or 6;
R₀ is selected from substituted or unsubstituted 5-8 membered heterocyclyl and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, C₁₋₆ haloalkyl, C₁₋₆ alkylhydroxyl, C₁₋₆ alkylamino, C₁₋₆ haloalkoxy, C₁₋₆ alkoxyhydroxyl, C₁₋₆ alkoxyamino, 3-6 membered cycloalkyl;
R₂, R₃, R₄, R₅, and R₆ are each independently selected from one or more of hydrogen, halogen, C₁₋₆ alkyl, amino, hydroxy, C₁₋₆ haloalkyl, C₁₋₆ alkylhydroxy, and C₁₋₆ alkylamino;
the heterocyclyl and heteroaryl and contain at least one heteroatom, and the heteroatom is selected from N, OorS.

The present invention also provides a histamine H3 receptor antagonist, include a series of compounds as numbered in BIOS-B-1 to BIOS-B-31, or isomers thereof, or solvates thereof, or pharmaceutically acceptable salts thereof:
BIOS-B-1: 2-morpholino-*N*-(4-(3-(piperidin-1-yl)cyclobutoxy)phenyl)acetamide;
BIOS-B-2: (*R*)-2-(2-methylpyrrolidin-1-yl)-*N*-(4-(3-(piperidin-1-yl)cyclobutoay)phenyl)acetamide;
BIOS-B-3: 2-(piperidin-1-yl)-*N*-(4-(3-(piperidin-1-yl)cyclobutoxy)phenyl)acetamide;
BIOS-B-4: 2-(4-methyl-1,4-diazepan-1-yl)-*N*-(4-(3-(piperidin-1-yl)cyclobutoxy)phenyl)acetamide;
BIOS-B-5: 2-(4,4-difluoropiperidin-1-yl)-*N*-(4-(3-(piperidin-1-yl)cyclobutoxy)phenyl)acetamide;
BIOS-B-6: 2-(4-hydroxypiperidin-1-yl)-*N*-(4-(3-(piperidin-1-yl)cyclobutoxy)phenyl)acetamide;
BIOS-B-7: *N*-(4-(3-(piperidin-1-yl)cyclobutoxy)phenyl)-2-thiomorpholinoacetamide;
BIOS-B-8: 2-(3-(2-hydroxyethyl)piperidin-1-yl)-*N*-(4-(3-(piperidin-1-yl)cyclobutoxy)phenyl)acetamide;
BIOS-B-9: 2-(piperazin-1-yl)-*N*-(4-(3-(piperidin-1-yl)cyclobutoxy)phenyl)acetamide;
BIOS-B-10: 2-(4-methylpiperazin-1-yl)-*N*-(4-(3-(piperidin-1-yl)cyclobutoxy)phenyl)acetamide;
BIOS-B-11: 2-(3-hydroxypiperidin-1-yl)-*N*-(4-(3-(piperidin-1-yl)cyclobutoxy)phenyl)acetamide;
BIOS-B-12: 2-(4-cyclobutylpiperazin-1-yl)-*N*-(4-(3-(piperidin-1-yl)cyclobutoxy)phenyl)acetamide;
BIOS-B-13:1-(2-morpholinoethyl)-3-(4-(3-(piperidin-1-yl)cyclobutoxy)phenyl)urea;
BIOS-B-14: (*R*)-1-(2-(2-methylpyrrolidin-1-yl)ethyl)-3-(4-(3-(piperidin-1-yl)cyclobutoxy)phenyl)urea;
BIOS-B-15: 1-(4-(3-(piperidin-1-yl)cyclobutoxy)phenyl)-3-(2-(piperidin-1-yl)ethyl)urea;
BIOS-B-16: 1-(2-(piperazin-1-yl)ethyl)-3-(4-(3-(piperidin-1-yl)cyclobutoxy)phenyl)urea;
BIOS-B-17: 1-(2-(4-methyl-1,4-diazepan-1-yl)ethyl)-3-(4-(3-(piperidin-1-yl)cyclobutoxy)phenyl)urea;
BIOS-B-18: 1-(2-(4,4-difluoropiperidin-1-yl)ethyl)-3-(4-(3-(piperidin-1-yl)cyclobutoxy)phenyl)urea;
BIOS-B-19: 1-(2-(4-hydroxypiperidin-1-yl)ethyl)-3-(4-(3-(piperidin-1-yl)cyclobutoxy)phenyl)urea;
BIOS-B-20: 1-(4-(3-(piperidin-1-yl)cyclobutoxy)phenyl)-3-(2-thiomorpholinoethyl)urea;
BIOS-B-21:1-(2-(2-(2-hydroxyethyl)piperidin-1-yl)ethyl)-3-(4-(3-(piperidin-1-yl)cyclobutoxy)phenyl) urea;
BIOS-B-22: 1-(2-(4-methylpiperazin-1-yl)ethyl)-3-(4-(3-(piperidin-1-yl)cyclobutoxy)phenyl)urea;
BIOS-B-23: 1-(2-(3-hydroxypiperidin-1-yl)ethyl)-3-(4-(3-(piperidin-1-yl)cyclobutoxy)phenyl)urea;
BIOS-B-24: 2-morpholino-*N*-(4-(3-(piperidin-1-yl)cyclobutoxy)phenyl)ethanethioamide;
BIOS-B-25: 2-morpholino-*N*-(4-(3-(piperidin-1-yl)cyclobutoxy)phenyl)ethanethioamide;
BIOS-B-26: (*R*)-2-(2-methylpyrrolidin-1-yl)-*N*-(4-(3-(piperidin-1-yl)cyclobutoxy)phenyl)ethanethioamide;
BIOS-B-27: 2-(piperidin-1-yl)-*N*-(4-(3-(piperidin-1-yl)cyclobutoxy)phenyl)ethanethioamide;
BIOS-B-28: 2-(4-methyl-1,4-diazepan-1-yl)-*N*-(4-(3-(piperidin-1-yl)cyclobutoxy)phenyl)ethanethioamide;
BIOS-B-29: 2-(diethylamino)-*N*-(4-(3-(piperidin-1-yl)cyclobutoxy)phenyl)acetamide;
BIOS-B-30: 2-(dimethylamino)-*N*-(4-(3-(piperidin-1-yl)cyclobutoxy)phenyl)acetamide; and
BIOS-B-31: 2-(2-(dimethylamino)ethoxy)-N-(4-(3-(piperidin-1-yl)cyclobutoxy)phenyl)acetamide.

The compound as numbered in BIOS-B-1 to BIOS-B-31, or pharmaceutically acceptable salts thereof, may have the following structural formula:

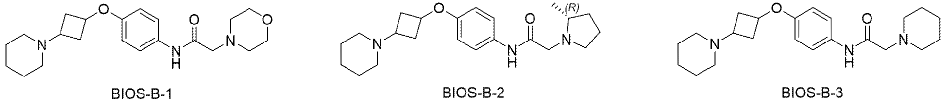

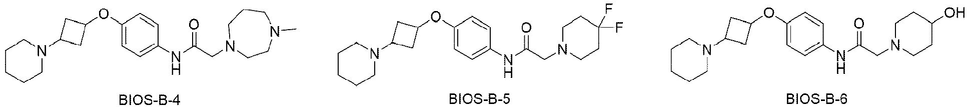

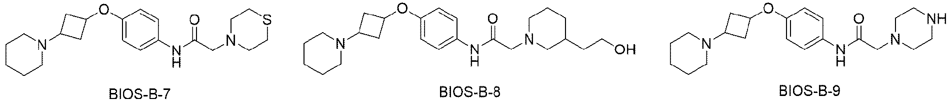

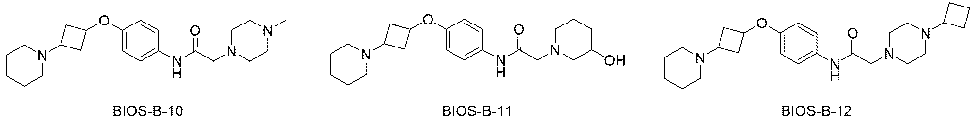

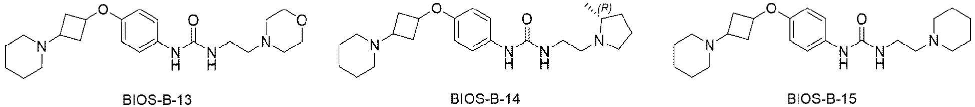

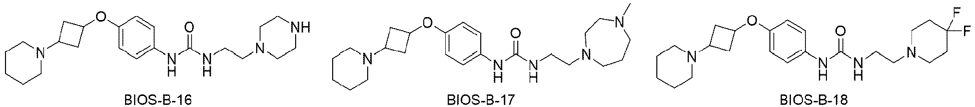

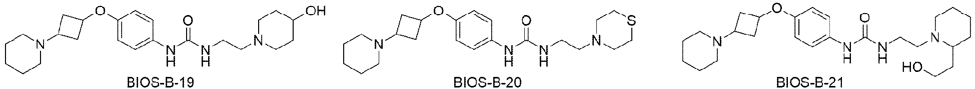

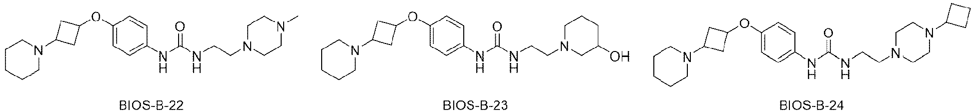

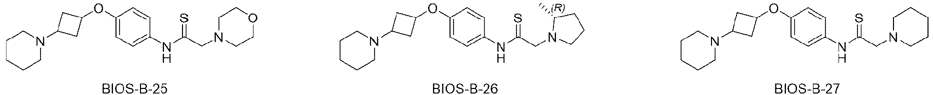

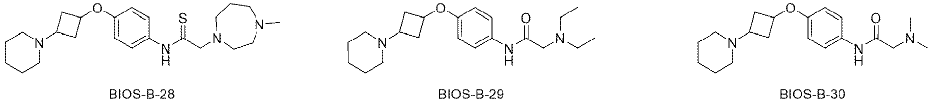

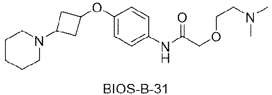

Furthermore, the present invention also provides a histamine H3 receptor antagonist, which is a chiral compound as follows or a pharmaceutically acceptable salt thereof:
(*R*)-2-(2-methylpyrrolidin-1-yl)-*N*-(4-(3-(piperidin-1-yl)cyclobutoxy)phenyl)acetamide;
(*R*)-1-(2-(2-methylpyrrolidin-1-yl)ethyl)-3-(4-(3-(piperidin-1-yl)cyclobutoxy)phenyl)urea; and
(*R*)-2-(2-methylpyrrolidin-1-yl)-*N*-(4-(3-(piperidin-1-yl)cyclobutoxy)phenyl)ethanethioamide.

The "compounds" described in the present invention include but are not limited to the following forms of compounds: free base, stereoisomers, geometric isomers, tautomers, isotopes, pharmaceutically acceptable salts, solvates, hydrates, prodrugs (esters or phosphates), etc.

The "compounds" described in the present invention can be asymmetric, for example, having one or more stereoisomers. Unless otherwise indicated, all stereoisomers are included, such as enantiomers and diastereomers. The compounds of the present invention containing asymmetric carbon atoms can be isolated in optically active pure form or in racemic form. Optically active pure forms can be obtained by splitting the racemic mixture, synthesizing it using chiral raw materials or chiral reagents.

The term "pharmaceutically acceptable salts" described in the present invention refers to salt of compounds in the present invention, prepared from compound has specific substituents with relatively non-toxic bases. When compounds contain relatively acidic functional groups, the bases addition salt of these compounds can be obtained by contacting the compound with a sufficient amount of base in pure solution or suitable inert solvents. Pharmaceutically acceptable bases addition salts, including, but not limited to, sodium, potassium, calcium, magnesium salts, ammonium or organic ammonia, such as alkali metal salts, alkaline earth metal salts, other metal salts, inorganic alkali salts, organic alkali salts, inorganic acid salts, lower alkane sulfonates, aryl sulfonates, organic acid salts, and amino acid salts.

In addition to the salt forms, the compounds provided herein exist in prodrug forms. The prodrugs of the compounds described herein are readily converted to the compounds of the present invention by chemical changes under physiological conditions. In addition, precursor drugs can be chemically or biochemically converted to the compounds of the present invention in vivo environment.

The compounds containing the above general structures, the terms used in this article have the following meanings:
The term "halogen" refers to fluorine, chlorine, bromine or iodine, preferably fluorine, chlorine or bromine.

The term "alkyl" refers to straight-chain or branched saturated hydrocarbon groups consisting of carbon atoms and hydrogen atoms, such as C₁₋₆ alkyl, including but not limited to methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2-methylhexyl.

The term "cycloalkyl" refers to monocyclic or bicyclic alkyl groups composed of carbon atoms and hydrogen atoms, such as a C₃₋₈ cycloalkyl group, including but not limited to cyclopropyl, cyclobutyl, cyclopentyl, cyclohexyl, cycloheptyl and cyclooctyl.

The term "alkoxy" refers to straight or branched alkyl groups connected by oxygen atoms, such as C₁₋₆ alkoxy, including but not limited to methoxy, ethoxy, n-propoxy (including n-propoxy and isopropoxy), butoxy (including n-butoxy, isobutoxy, sec-butoxy or tert-butoxy), pentoxy (including n-pentoxy, isopentoxy, neopentoxy), n-hexyloxy, 2-methylhexyloxy, etc.

The term "alkylamino" refers to open-chain alkyl groups containing nitrogen atoms, such as C₁₋₆ alkylamino, including but not limited to methylamino, ethylamino, isopropylamino, dimethylamino, methylethylamino, diethylamino, etc.

The term "aryl" refers to all-carbon monocyclic or condensed polycyclic groups of 5-12 (integer) carbon atoms, having a completely conjugated π-electron system, including but not limited to benzene rings, naphthalene rings, and anthracene rings.

The term "heterocyclic group" or "heteroaryl" refers to monocyclic or condensed rings having 3-12 (integer) ring atoms, wherein 1, 2, 3 or more ring atoms are one or more selected from N, O, and S, and the remaining ring atoms are C, and have a completely conjugated or unconjugated π-electron system. The heterocyclic group can be saturated or unsaturated groups. Examples of heterocyclic groups include, but are not limited to, pyrrolyl, indolyl, pyrrolidinyl, imidazolyl, pyrazolyl, tetrazolyl, pyridinyl, quinolyl, isoquinolyl, piperidinyl, pyrimidinyl, pyrazinyl, piperazinyl, furanyl, pyranyl, and morpholinyl.

The present provides a method of preparing the above compounds by the following steps, but not limited to the following methods:
**General synthesis method 1: Refer to general formula II, where X** = **O, R₁ = H, R₀ = NR'R"**

The reaction route is as follows:

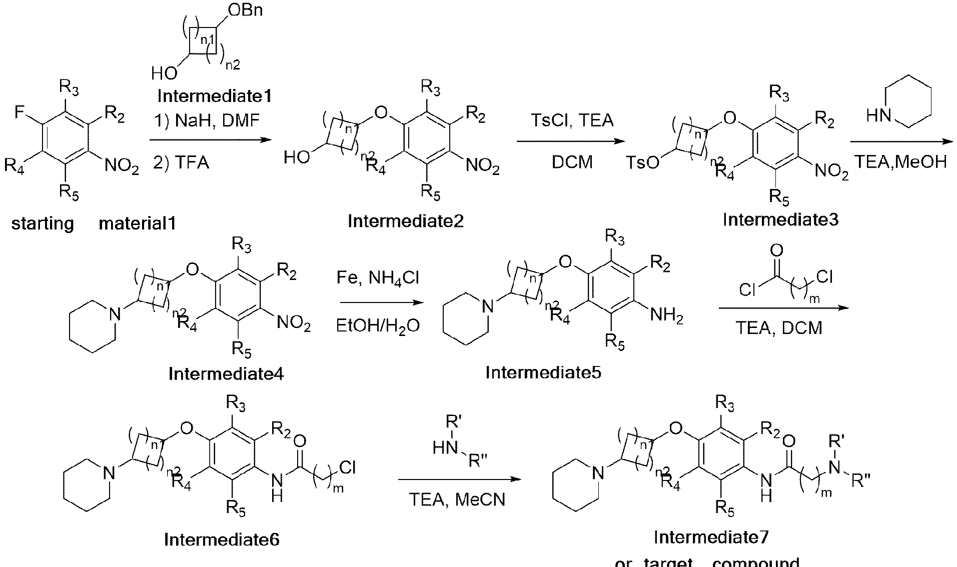

The corresponding reaction procedure is shown as follows:
The starting material 1 (p-fluoronitrobenzene analogs) reacted with Intermediate 1 and sodium hydride dissolved in *N*,*N*-dimethylformamide, and then reacted in trifluoroacetic acid to obtain intermediate 2. Intermediate 2 then reacted with p-toluenesulfonyl chloride to obtain intermediate 3. Intermediate 3 reacted with piperidine via SN₂ to obtain intermediate 4. Intermediate 4 was reduced to intermediate 5 using ammonium chloride as hydrogen source under Fe catalysis. Intermediate 5 was acylated by halogenated alkyl chloride to intermediate 6, which underwent reductive amination to obtain intermediate 7 or the target compounds.

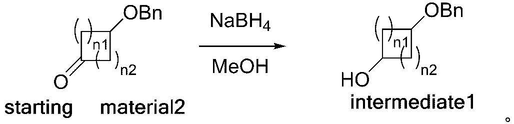

The corresponding preparation route of Intermediate 1 is shown as follows:
General synthesis method 2: Refer to general formula III, where X=O, R₁=H, R₆=H, R₀ =NR'R"

The corresponding preparation route is shown as follows:

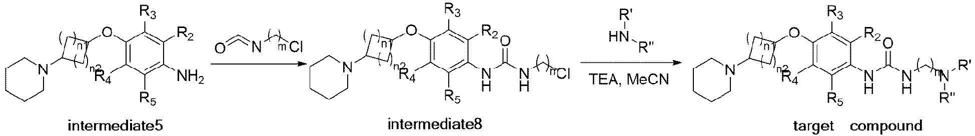

The corresponding preparation process is shown as follows:
Intermediate 5 reacted with chloroalkyl isocyanate to form intermediate 8, which is the substituted with amineto afford the target compounds.

### General synthesis method 3: Refer to general formula II, where X = S, R₁ = H, R₀ = NR'R"

The corresponding preparation route is shown as follows:

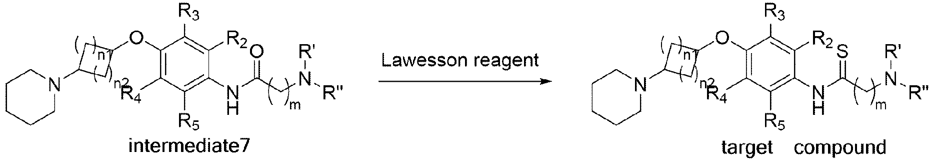

The reaction process is as follows: intermediate 7 is reacted with Lawesson's reagent to obtain the target compounds.

The substituents R', R", R₁, R₂, R₃, R₄, and R₅ involved in the aforementioned synthesis method 1, synthesis method 2, and synthesis method 3 are defined as follows:
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxyl, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time; R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, and C₁₋₆ alkylsulfoxide; or, R₂ and R₃ together with the carbon atoms on the benzene ring to which they are attached form a substituted or unsubstituted benzobicyclic structure, wherein the benzobicyclic structure can be but not limited to, a benzoheterocyclic ring; or, R₄ and R₅ together with the carbon atoms on the benzene ring to which they are attached form a substituted or unsubstituted benzobicyclic structure, wherein the benzobicyclic structure can be but not limited to, a benzoheterocyclic ring .

The present invention also provides a pharmaceutical composition comprising at least one compound as described above or pharmaceutically acceptable salt thereof as active ingredient, and at least one or more pharmaceutically acceptable carriers.

The term "pharmaceutical composition" as used in the invention refers to a formulation of one or more compounds of the present invention or salts thereof with a carrier commonly accepted in the art for the delivery of biologically active compounds to an organism (e.g., human beings). The pharmaceutical compositions are intended to facilitate drug delivery to the organism.

The routes of administration of the compounds or pharmaceutically acceptable salts thereof, or pharmaceutical compositions thereof described herein, include, but are not limited to, oral, rectal, transmucosal, trans-intestinal, topical, transdermal, inhalation, parenteral, sublingual, intravaginal, intranasal, intraocular, intraperitoneal, intramuscular, subcutaneous, and intraventricular administration. The preferred route of administration is oral administration.

The present invention also provides uses of preparing compounds or pharmaceutical compositions as described hereinbefore in the prevention or treatment of diseases associated with histamine H3 receptors.

Preferably, the foregoing medications are used for the prevention or treatment of cognitive disorders, dementia, attention deficit hyperactivity disorder, schizophrenia, epilepsy, sleep disorders, sleep apnea, obesity, eating disorders, pain or pruritus.

Preferably, the foregoing medications are used for the prevention or treatment of neuropathic pain, which can be but not limited to, peripheral neuropathic pain or central neuropathic pain.

Preferably, the peripheral neuropathic pain is trigeminal neuralgia, glossopharyngeal neuralgia, acute or chronic inflammatory demyelinating polyneuropathic neuralgia, alcoholic polyneuralgia, chemotherapy-induced polyneuropathic neuralgia, complex regional pain syndrome, embedded compression neuralgia (e.g., carpal tunnel syndrome), HIV sensory neuralgia, medically induced neuralgia (e.g., post-mastectomy pain), tumor compression or infiltration neuralgia, nutritional deficiency-associated neuralgia, diabetic neuralgia, phantom limb pain, postherpetic neuralgia, post-radiation plexopathy, radiculopathy (cervical, thoracic, or lumbosacral), toxic exposure-associated neuralgia, or post-traumatic neuralgia.

Preferably, the central neuropathic pain is post-stroke pain, multiple sclerosis-associated pain, Parkinson's disease-associated pain, post-traumatic spinal cord injurious pain, spinal cord cavernous disease, post ischemic myelopathy, compressive myelopathy, HIV myelopathy, or post-radiation myelopathy.

Compared with the prior arts, the present invention has the following beneficial effects:
The present invention has developed a series of structurally novel histamine H3 receptor antagonists based on histamine H3 receptor ligands. The series of compounds are characterized by simple preparation routes and easy availability of raw materials. The results of the series of related biological tests all indicate that the compounds have significant H3 receptor antagonistic activity and can be used as lead compounds to prevent or treat a disease associated with histamine H3 receptor.

### DESCRIPTION OF THE DRAWINGS

Figure 1 shows the graphs of the changes in mechanical withdrawal thresholds of each experimental group at different times and different doses. In the graph, compared with the MODEL group, *, P<0.05; **, P<0.01; compared with the SHAM group, ^{#}, P<0.05; ^{##}, P<0.01.
Figure 2 shows the change of mechanical withdrawal thresholds of each experimental group at different times. In the graph, compared with the MODEL group, *, P<0.05; **, P<0.01; compared with the SHAM group, ^{#}, P<0.05; ^{##}, P<0.01.

### EMBODIMENTS OF THE INVENTION

The following are specific embodiments of the present invention to further describe the technical solution, but the protection scope of the present invention is not limited to these embodiments. Any changes or equivalent substitutions that do not deviate from the concept of the present invention are included in the protection scope of the present invention.

The solvents, equipment, codes and full names involved in the embodiments of the present invention are as follows:
Code Bn: refers to benzyl;
Code DMF: refers to *N*,*N*-dimethylformamide;
Code TFA: refers to trifluoroacetic acid;
Code TsCl: refers to *p*-Toluenesulfonyl chloride;
Code TEA: refers to triethylamine;
Code DCM: refers to dichloromethane;
Code Boc: refers to tert-butyloxycarbonyl;
Code Lawesson reagent: 2,4-bis(p-methoxyphenyl)-1,3-dithiodiphosphatane-2,4-sulfide.

In the methods for preparing the target compounds provided by the present invention: the liquid chromatography uses Waters Symmetry C18 chromatographic columns; TLC is performed using GF254 (0.25 mm); Nuclear magnetic resonance spectroscopy (NMR) is measured by Bruker-400 NMR spectrometers; Liquid chromatography-mass spectrometry (LC/MS) is performed by Waters ZQ mass spectrometer detector (column: Waters Symmetry C18, mm, 5 µm, 35 °C) in ESI (+) ion mode.

In addition, all operations involving easily oxidized or hydrolyzed starting materials are carried out under nitrogen protection. Unless otherwise specified, the raw materials used in the present invention are commercially available and can be used directly without further purification.

The starting materials, common intermediates, etc. involved in the embodiments of the present invention can be purchased commercially or obtained by self-production. Among them, the starting materials and common intermediates that need to be obtained by self-production are prepared in detail as follows.

### I. Preparation of starting material 2:

The starting material 2 involved in the preparation process of the common intermediate material B1 was synthesized by the applicant, and the synthesis route is as follows:

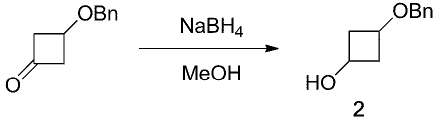

To a 250 mL bottle added 3-(benzyloxy)cyclobutanone (5.0 g, 28.37 mmol) and anhydrous methanol (34 mL) under Nitrgen atmosphere. The reaction was cooled by ice for 15 minutes before sodium borohydride added in batches (717 mg/batceh, 3 batches, 56.75 mmol) during 0.5 hours, and finnaly the reaction was transferred to room temperature for 4-5 hours. The reaction of the raw material was complete as detected by thin layer chromatography.

The reaction solution was quenched with 1.0 mL of saline, and sepatated with ethyl acetate twice. The organic phase was combined and washed by saline, dried over by anhydrous sodium sulfate, and finally concentrated to dryness obtaining 5.31 g of colorless oily liquid **(compound 2)** yielding 99%.
MS:179.1[M+H]+_{∘}
¹H NMR (400 MHz, DMSO-*d*₆) δ 7.38~7.26 (m, 5H), 4.48 (s, 2H), 4.04 (t, *J =* 5.6 Hz, 1H), 3.92~3.79 (m, 1H), 3.80 (d, *J =* 4.4 Hz, 1H), 2.35~2.30 (m 2H), 2.03~1.99 (m, 2H).

### II. Preparation of common Intermediate B1:

The synthesis route of **common** intermediate B1 is as follows:

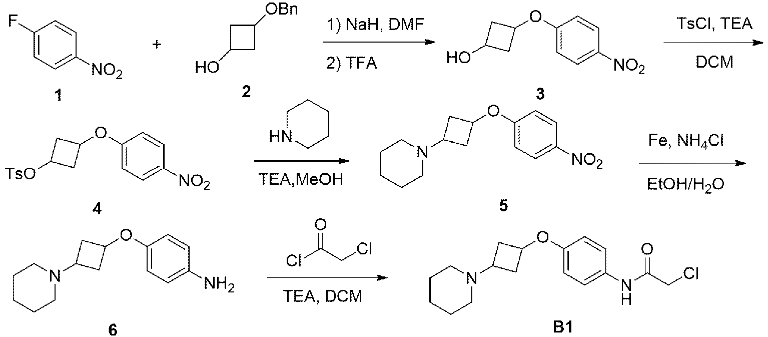

### Step 1:Synthesis of 3-(4-nitrophenoxy)cyclobutanol (3)

Compound 2 (2.155 g, 12.09 mmol) was dissolved in 15 mL anhydrous N,N-dimethylformamide, and cooled by ice for 5 minutes. Sodium hydride (580 mg, 14.51 mmol) was added under nitrogen atmosphere and reacted in ice bath for 0.5-1 hour. A solution of 4-fluoronitrobenzene (compound 1, 1.54 g) in 5 mL anhydrous N,N-dimethylformamide was added slowly dropwise within 15 minutes. The reaction was carried out at room temperature for 5-6 hours. The reaction of the raw material was complete as detected by thin layer chromatography.

The reaction solution was quenched by 200 mL ice water, extracted by ethyl acetate (50 mL × 3), the organic phase was combined, washed by water, saline, and dried with anhydrous sodium sulfate, concentrated to obtain 3.89 g yellow crude product yielding 99%.

The yellow crude (3.89 g) was dissolved in 40 mL trifluoroacetic acid and reluxed for 3-4 hours. When the reaction was completed, the reaction was cooled to 0 °C by ice water bath. Sodium hydroxide solution was added dropwise to adjust the pH to neutral, extracted by ethyl acetate (70 mL × 3), and the organic phases were combined. The organic phase was washed by saline, dried over anhydrous sodium sulfate, and concentrated to obtain 4.4 g crude yellow produce. Rapid column chromatography purification (PE:EA = 2:1) yielded 1.358 g light yellow powder **(compound 3)** with a yield of 53.7%.
MS:210.1[M+H]⁺_{∘}
¹H NMR (400 MHz, DMSO-*d*₆) δ 8.19 (d, *J* = 9.2 Hz, 2H), 7.05 (d, *J* = 9.2 Hz, 2H), 5.26 (d, *J* = 6.8 Hz, 1H), 4.43 (m, 1H), 3.87 (m, 1H), 2.91~2.83 (m, 2H), 2.01~1.87 (m, 2H)_{∘}

### Step 2:Synthesis of 3-(4-nitrophenoxy)cyclobutyl 4-methylbenzenesulfonate (4)

Compound 3 (4.64 g, 22.22 mmol), 9.2 mL triethylamine and p-toluenesulfonic chloride (1.86 g, 33.33 mmol) were dissolved in 40 mL anhydrous dichloromethane under nitrogen atmosphere. The mixture was transferred to oil bath and reacted overnight under 30 °C. The reaction of the raw material was complete as detected by thin layer chromatography.

The reaction solution was quenched with 20 mL water, the aqueous phase was extracted with dichloromethane (50 mL × 3). The organic phase was combined, washed with saline, dried with anhydrous sodium sulfate, and concentrated to obtain the crude product. Finally, the crude was purified and crystallized by methyl tert butyl ether obtaining 6.73 g white powder **(compound 4)** with a yield of 83.38%.
MS:364.1[M+H]⁺_{∘}
¹H NMR (400 MHz, DMSO-*d*₆) δ 8.17 (s, 2H), 7.82 (d, *J =* 9.0 Hz, 2H), 7.50 (d, *J =* 9.0 Hz, 2H), 7.00 (d, *J* = 7.8, 2H), 4.62 (t, *J* = 7.0 Hz, 1H), 4.50 (t, *J* = 7.0 Hz, 1H), 2.94~2.87 (m, 2H), 2.43 (s, 3H), 2.22~2.15 (m, 2H)_{∘}

### Step 3:Synthesis of 1-(3-(4-nitrophenoxy)cyclobutyl)piperidine (5)

Compound 4 (800 mg, 2.20 mmol) was mixed with 10 mL piperidine, reacted overnight in 100 °C oil bath. The reaction of the raw material was complete as detected by thin layer chromatography.

The reaction solution was cooled to room temperature, quenched with 20 mL water, and then extracted with ethyl acetate (50 mL × 3). The organic phase was combined and washed with saline, dried over with anhydrous sodium sulfate, and concentrated to obtain 736 mg crude **(compound 5),** yielding 99%.
MS:277.1[M+H]⁺.
¹H NMR (400 MHz, DMSO-*d*₆) δ 8.28~8.14 (d, 2H), 7.11~6.98 (d, 2H), 4.90~4.85 (m, 1H), 2.91~2.86 (m, 1H), 2.91~2.86 (m, 3H), 2.28~2.14 (m, 5H), 1.55~1.30 (m, 10H)_{∘}

### Step 4:Synthesis of 4-(3-(piperidin-1-yl)cyclobutoxy)aniline (6)

Compound 5 (736 mg), reduced iron powder (862 mg, 7.0 equiv.) and ammonium chloride (214 mg, 3.96 mmol) were dissolved in 6 mL ethanol and 2 mL water. The reaction was carried out at 80 °C oil bath for 1-2 hours. The reaction of the raw material was complete as detected by thin layer chromatography.

The reaction solution was cooled to roomtemperature.The reaction solution was filtered through diatomaceous earth and the residue was washed with ethanol.The filtrate was concentrated to dryness, and 10 mL sodium bicarbonate solution was added, and the extracted with ethyl acetate (20 mL × 3). The organic phase was combined, washed with saline, dried over with anhydrous sodium sulfate, and concentrated to obtain 583 mg brown liquid **(compound 6),** yielding 99%.
MS:347.2[M+H]⁺_{∘}

### Step 5: Synthesis of 2-chloro-N-(4-(3-(piperidin-1-yl)cyclobutoxy)phenyl)acetamide (Bl)

Compound 6 (852 mg, 3.46 mmol) and triethylamine (961 µL, 6.92 mmol) were dissolved in 12 mL anhydrous dichloromethane. 358 µL chloroacetyl chloride was added and allowed to react overnight at room temperature. The reaction of the raw material was complete as detected by thin layer chromatography.

The reaction solution was quenched with 15 mL sodium bicarbonate solution, extracted with dichloromethane (20 mL × 3). The organic phase was combined and washed with saline, dried with anhydrous sodium sulfate, and concentrated to obtain 1.42 g crude. Rapid column chromatography purification (PE:EA:TEA = 2:1:0.2) yielded 720 mg light yellow powder **(compound B1),** yielding 64.5%.
MS: 352.3[M+H]⁺_{∘}
¹H NMR (400 MHz, DMSO-*d*₆) δ 10.14 (s, 1H), 7.47 (d, 2H), 6.78 (d, 2H), 4.70~4.65 (m, 1H), 4.21 (s, 2H), 2.91~2.79 (m, 1H), 2.37~2.05 (m, 8H), 1.55~1.32 (m, 6H).

### III. Preparation of common Intermediate B2:

The synthesis route of intermediate material B2 is as follows:

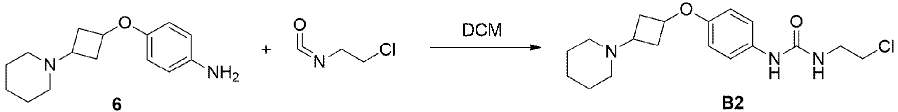

Compound 6 (1.896 g, 7.70 mmol) was dissolved in 30 mL Anhydrous dichloromethane. 788 µL chloroehyl isocyanate was added at -5 °C, and the reaction was finally transferred to room temperature for 5-6 hours (with large amount of white precipitate appeared). The reaction of the raw material was complete as detected by thin layer chromatography.

The reaction solution was filtered, and the residue was washed with a small amount of dichloromethane, and the residue was dried, affording 1.894 g white powder **(compound B2),** yielding 73.3%.
MS:352.3[M+H]⁺_{∘}
¹H NMR (400 MHz, DMSO-*d*₆) δ 8.42 (s, 1H), 7.26 (d, *J =* 9.0 Hz, 2H), 6.69 (d, *J =* 9.0 Hz, 2H), 6.28 (t, *J* = 5.8 Hz, 1H), 4.66~4.61 (m, 1H), 3.64 (t, *J* = 6.2 Hz, 2H), 3.40 (q, *J* = 6.2 Hz, 2H), 2.85 (s, 1H), 2.40~1.98 (m, 8H), 1.60~1.25 (m, 6H).

The following Example 1 to 31 are the preparation examples of the free base and/or hydrochloride structure of the BIOS-S-series compounds described in the present invention. It should be noted that in addition to the hydrochloride from mentioned in the Examples, BIOS-B series compounds can also be prepared in other pharmaceutically acceptable salt forms, including but not limited to sulfates, nitrates, nitrites, maleic acid salts, fumarates, formatted, diformes, and acetates, etc. The preparation of these compounds can be inspired by the corresponging technical insights provided in Examples 1 to 31 of the present invention.

### Example 1: Preparation of 2-morpholino-N-(4-(3-(piperidin-1-yl)cyclobutoxy)phenyl)acetamid e (BIOS-B-1)

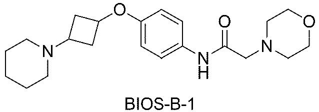

The synthesis route is as follows:

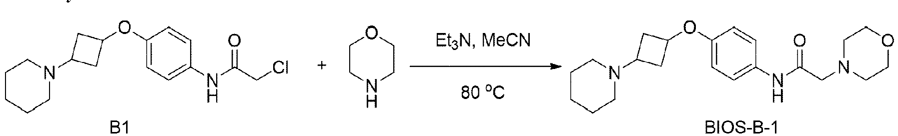

Intermediate B1 (100 mg, 0.310 mmol), morpholine (81 µL, 0.929 mmol), and triethylamine 323 µL were dissolved in 4 mL acetonitrile and transferred to react overnight at 80 °C. The reaction of the raw material was complete as detected by thin layer chromatography.

The reaction solution was concentrated to dryness and the crude product was purified on a preparative plate (petroleum ether: ethyl acetate: triethylamine = 5: 4: 1), affording 81 mg light brown oily liquid **(compound BIOS-B-1),** yielding 69.96%.

Furthermore, the hydrochloride of compound BIOS-B-1 could be prepared by reacted with HCl/EA for 1-2 hours, which afford 86 mg white solid with a yield of 88.83%.
MS: 374.1[M+H]⁺_{∘}
¹H NMR (400 MHz, DMSO-*d*₆) δ 9.58 (s, 1H), 7.50 (d, *J* = 9.0 Hz, 2H), 6.75 (d, *J* = 9.0 Hz, 2H), 4.69~4.64 (m, 1H), 3.70~3.54 (m, 4H), 3.08 (s, 2H), 2.88~2.81 (m, 1H), 2.50~2.46 (m, 4H), 2.37~2.00 (m, 8H), 1.60~1.35 (m, 6H).

### Example 2: Preparation of (R)-2-(2-methylpyrrolidin-1-yl)-N-(4-(3-(piperidin-1-yl)cyclobutoxy) phenyl)acetamide (BIOS-B-2)

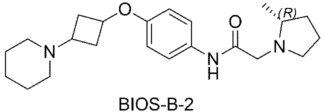

The synthesis route is as follows:

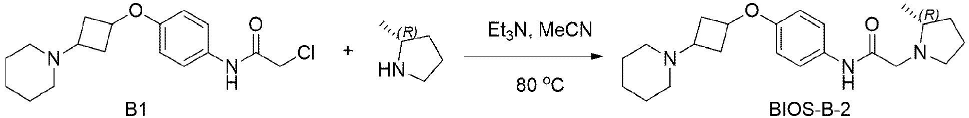

Referred to the synthesis route of compound BIOS-B-1 in Example 1, in this example, (R)-2-methylpyrrolidine from this example was used instead of morpholine from Example 1, and the remaining preparation steps were the same as in Example 1.75 mg yellow brown oily liquid **(compound BIOS-B-2)** was obtained with a yield of 65.12%.

Furthermore, the hydrochloride of compound BIOS-B-2 could be prepared by reacted with HCl/EA for 1-2 hours, which afford 71 mg pink solid with a yield of 79.13%.
MS : 372.1[M+H]⁺_{∘}
¹H NMR (400 MHz, DMSO-*d*₆) δ 9.47 (s, 1H), 7.50 (d, *J* = 9.0 Hz, 2H), 6.75 (d, *J* = 9.0 Hz, 2H), 4.69~4.64 (m, 1H), 3.38 (d, *J =* 15.5 Hz, 1H), 3.11~3.06 (m, 1H), 2.95 (d, *J =* 15.5 Hz, 1H), 2.88~2.81 (m, 1H), 2.49~2.03 (m, 10H), 1.97~1.87 (m, 1H), 1.81~1.60 (m, 2H), 1.57~1.29 (m, 7H), 1.06 (d, *J =* 6.1 Hz, 3H).

### Example 3: Preparation of 2-(piperidin-1-yl)-N-(4-(3-(piperidin-1-yl)cyclobutoxy)phenyl)acetamide (BIOS-B-3)

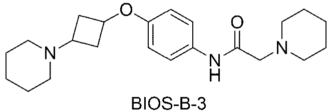

The synthesis route is as follows:

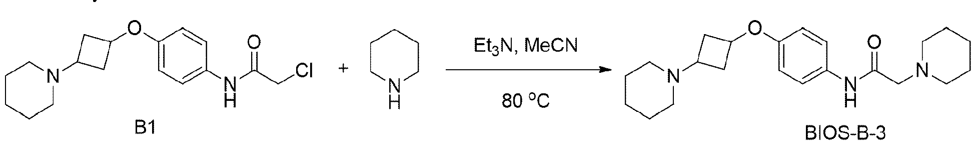

Intermediate B1 (100 mg, 0.310 mmol), morpholine (81 µL, 0.929 mmol), and triethylamine 323 µL were dissolved in 4 mL acetonitrile and transferred to react overnight in 80 °C oil bath. The reaction of the raw material was complete as detected by thin layer chromatography.

The reaction solution was concentrated to dryness and the crude product was purified on a preparative plate (petroleum ether: ethyl acetate: triethylamine = 5: 4: 1), affording 100 mg light yellow solid **(compound BIOS-B-3),** yielding 86.82%.
MS: 372.1[M+H]⁺_{∘}
¹H NMR (400 MHz, DMSO-*d*₆) δ 9.50 (s, 1H), 7.50 (d, *J* = 9.0 Hz, 2H), 6.75 (d, *J* = 9.0 Hz, 2H), 4.70~4.65 (m, 1H), 3.02 (s, 2H), 2.95~2.81 (m, 1H), 2.44 (t, *J =* 5.3 Hz, 4H), 2.38~2.03 (m, 8H), 1.66~1.31 (m, 12H).

### Example 4: Preparations of 2-(4-methyl-1,4-diazepan-1-yl)-N-(4-(3-(piperidin-1-yl)cyclobutoxy) phenyl)acetamide (BIOS-B-4)

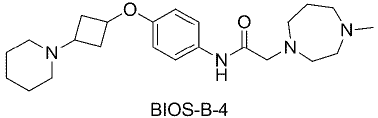

The synthesis route is as follows:

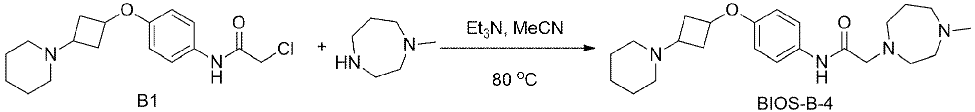

Referred to the synthesis route of compound BIOS-B-1 in Example 1, in this example, 1-methyl-1,4-diazepane from this example was used instead of morpholine from Example 1, and the remaining preparation steps were the same as in Example 1.92 mg yellow solid **(compound BIOS-B-4)** was obtained with a yield of 74.09%.

Furthermore, the hydrochloride of compound BIOS-B-4 could be prepared by reacted with HCl/EA for 1-2 hours, which afford 76 mg light yellow solid with a yield of 64.89%.
MS:401.1[M+H]⁺_{∘}
¹H NMR (400 MHz, DMSO-*d*₆) δ 9.53 (s, 1H), 7.50 (d, *J* = 9.0 Hz, 2H), 6.75 (d, *J* = 9.0 Hz, 2H), 4.70~4.64 (m, 1H), 3.23 (s, 2H), 2.87~2.83 (m, 1H), 2.80~2.71 (m, 4H), 2.64~2.56 (m, 4H), 2.38~2.14 (m, 9H), 2.12~2.06 (m, 2H), 1.83~1.71 (m, 2H), 1.56~1.32 (m, 6H).

### Example 5: Preparation of 2-(4,4-difluoropiperidin-1-yl)-N-(4-(3-(piperidin-1-yl)cyclobutoxy)p henyl)acetamide (BIOS-B-5)

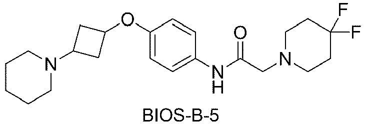

The synthesis route is as follows:

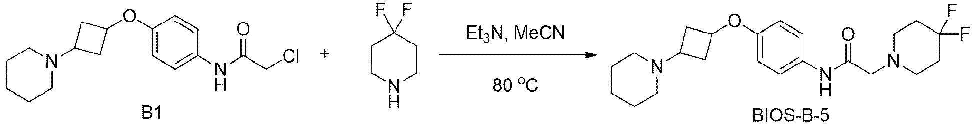

Referred to the synthesis route of compound BIOS-B-3 in Example 3, in this example, 4,4-difluoropiperidine from this example was used instead of piperidine from Example 3, and the remaining preparation steps were the same as in Example 3.61 mg light yellow solid **(compound BIOS-B-5)** was obtained with a yield of 69.66%.
MS:408.1[M+H]⁺_{∘}
¹H NMR (400 MHz, DMSO-*d*₆) δ 9.59 (s, 1H), 7.51 (d, *J* = 9.0 Hz, 1H), 6.75 (d, *J* = 9.0 Hz, 2H), 4.70~4.65 (m, 1H), 3.17 (s, 2H), 2.87 (s, 1H), 2.64 (t, *J =* 5.6 Hz, 4H), 2.40~1.95 (m, 12H), 1.57~1.31 (m, 6H).

### Example 6: Preparation of 2-(4-hydroxypiperidin-1-yl)-N-(4-(3-(piperidin-1-yl)cyclobutoxy)ph enyl)acetamide (BIOS-B-6)

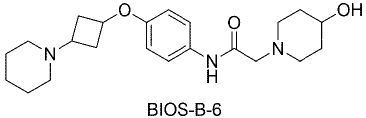

The synthesis route is as follows:

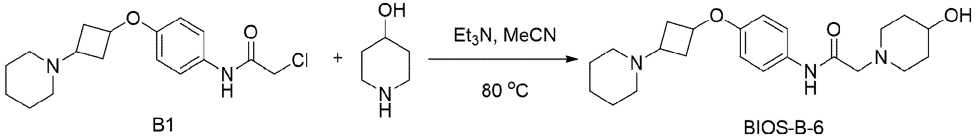

Referred to the synthesis route of compound BIOS-B-1 in Example 1, in this example, 4-hydroxypyridine from this example was used instead of morpholine from Example 1, and the remaining preparation steps were the same as in Example 1.94 mg yellow solid **(compound BIOS-B-6)** was obtained with a yield of 78.25%.

Furthermore, the hydrochloride of compound BIOS-B-6 could be prepared by reacted with HCl/EA for 1-2 hours, which afford 99 mg white solid with a yield of 88.64%.
MS: 388.1[M+H]⁺_{∘}
¹H NMR (400 MHz, DMSO-*d*₆) δ 9.51 (s, 1H), 7.50 (d, *J* = 9.0 Hz, 2H), 6.75 (d, *J* = 9.0 Hz, 2H), 4.70~4.65 (m, 1H), 4.58 (d, *J =* 4.0 Hz, 1H), 3.50~3.45 (m, 1H), 3.04 (s, 2H), 2.91 (s, 1H), 2.76~2.71 (m, 2H), 2.40~2.03 (m, 10H), 1.80~1.68 (m, 2H), 1.60~1.32 (m, 8H).

### Example 7: Preparation of N-(4-(3-(piperidin-1-yl)cyclobutoxy)phenyl)-2-thiomorpholinoacetamide (BIOS-B-7)

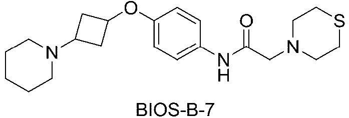

The synthesis route is as follows:

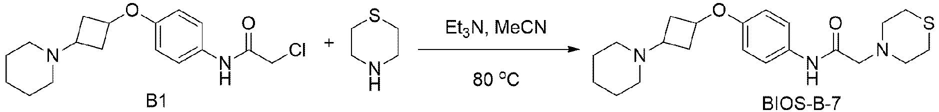

Referred to the synthesis route of compound BIOS-B-3 in Example 3, in this example, thiomorpholine from this example was used instead of piperidine from Example 3, and the remaining preparation steps were the same as in Example 3.86 mg light yellow solid **(compound BIOS-B-7)** was obtained with a yield of 71.21%.
MS: 390.0[M+H]⁺_{∘}
¹H NMR (400 MHz, DMSO-*d*₆) δ 9.51 (s, 1H), 7.51 (d, *J* = 9.0 Hz, 2H), 6.75 (d, *J* = 9.0 Hz, 2H), 4.70~4.65 (m, 1H), 3.10 (s, 2H), 2.87 (s, 1H), 2.77~2.74 (m, 4H), 2.73~2.63 (m, 4H), 2.40~2.04 (m, 8H), 1.61~1.32 (m, 6H).

### Example 8: Preparation of 2-(3-(2-hydroxyethyl)piperidin-1-yl)-N-(4-(3-(piperidin-1-yl)cyclob utoxy)phenyl)acetamide (BIOS-B-8)

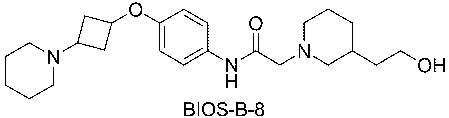

The synthesis route is as follows:

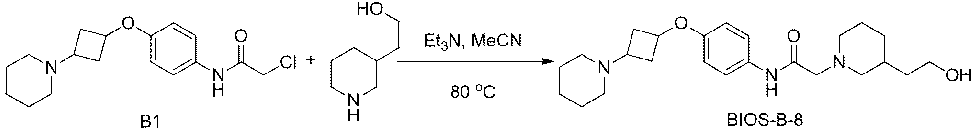

Referred to the synthesis route of compound BIOS-B-1 in Example 1, in this example, 2-piperidine ethanol from this example was used instead of morpholine from Example 1, and the remaining preparation steps were the same as in Example 1. 105 mg colorless oily liquid **(compound BIOS-B-8)** was obtained with a yield of 81.50%.

Furthermore, the hydrochloride of compound BIOS-B-8 could be prepared by reacted with HCl/EA for 1-2 hours, which afford 101 mg white solid with a yield of 81.83%.
MS:416.1[M+H]⁺_{∘}
¹H NMR (400 MHz, DMSO-*d*₆) δ 9.53 (s, 1H), 7.50 (d, *J* = 9.1 Hz, 1H), 6.76 (d, *J* = 9.1 Hz, 2H), 4.70~4.65 (m, 1H), 4.46 (s, 1H), 3.57~3.38 (m, 2H), 3.26 (d, *J =* 16.4 Hz, 1H), 3.05 (d, *J =* 16.4 Hz, 1H), 2.93~2.70 (m, 2H), 2.59~2.55 (m, 1H), 2.46~2.16 (m, 7H), 2.13~2.08 (m, 2H), 1.77~1.62 (m, 3H H), 1.54~1.45 (m, 7H), 1.44~1.26 (m, 4H).

### Example 9: Preparation of 2-(piperazin-1-yl)-N-(4-(3-(piperidin-1-yl)cyclobutoxy)phenyl)aceta mide (BIOS-B-9)

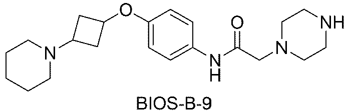

The synthesis route is as follows:

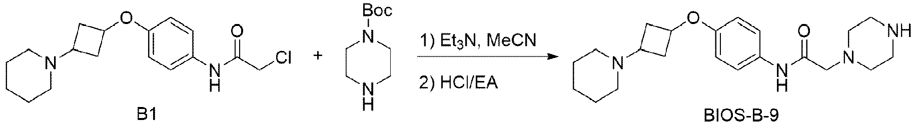

Intermediate B1 (150 mg, 0.465 mmol), 1-Boc-piperazine (260 mg, 1.394 mmol), and 323 µL triethylamine were dissolved in 4 mL acetonitrile and transferred to reaction overnight in 80 °C oil bath. The reaction of the raw material was complete as detected by thin layer chromatography.

The reaction solution was concentrated to dryness and the crude product was purified on a preparative plate (petroleum ether: ethyl acetate: triethylamine = 5: 4: 1) , affording 179 mg pale white solid intermediate. 105 mg of the intermediate was reacted with HCl/EA for 1-2 hour, affording 105 mg white solid **(the hydrochloride of compound BIOS-B-9),** with a two-step yield of 79.89%.
MS:373.1[M+H]⁺.
¹H NMR (400 MHz, DMSO-*d*₆) δ 11.27 (s, 1H), 10.72 (s, 1H), 9.82 (s, 2H), 7.56 (d, *J =* 9.0 Hz, 2H), 6.83 (d, *J* = 9.0 Hz, 2H), 4.92~4.87 (m, 1H), 4.10 (s, 2H), 3.83 (d, *J* = 7.9 Hz, 1H), 3.58~3.32 (m, 8H), 2.96~2.89 (m, 2H), 2.73~2.64 (m, 2H), 2.40~2.29 (m, 2H), 1.94~1.65 (m, 5H), 1.40~1.30 (m, 1H).

### Example 10: Preparation of 2-(4-methylpiperazin-1-yl)-N-(4-(3-(piperidin-1-yl)cyclobutoxy)ph enyl)acetamide (BIOS-B-10)

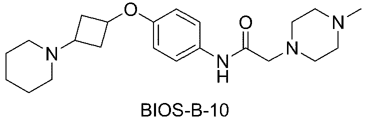

The synthesis route is as follows:

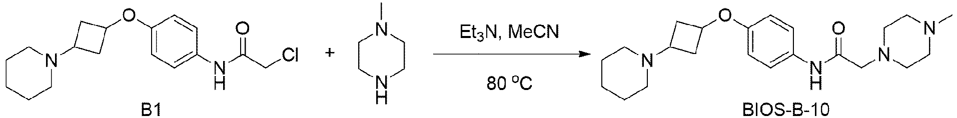

Referred to the synthesis route of compound BIOS-B-3 in Example 3, in this example, 4-methylpiperazine from this example was used instead of piperidine from Example 3, and the remaining preparation steps were the same as in Example 3. 107 mg pale yellow solid (compound BIOS-B-10) was obtained with a yield of 89.30%.
MS:373.1[M+H]⁺_{∘}
¹H NMR (400 MHz, DMSO-*d*₆) δ 9.51 (s, 1H), 7.49 (d, *J* = 9.0 Hz, 2H), 6.75 (d, *J* = 9.0 Hz, 2H), 4.70~4.65 (m, 1H), 3.06 (s, 2H), 2.86 (d, *J =* 7.8 Hz, 1H), 2.42~2.20 (m, 14H), 2.18 (s, 3H), 2.13~2.07 (m, 2H), 1.55~1.32 (m, 6H).

### Example 11: Preparation of 2-(3-hydroxypiperidin-1-yl)-N-(4-(3-(piperidin-1-yl)cyclobutoxy)p henyl)acetamide (BIOS-B-11)

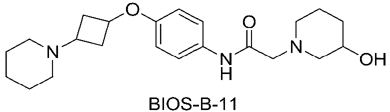

The synthesis route is as follows:

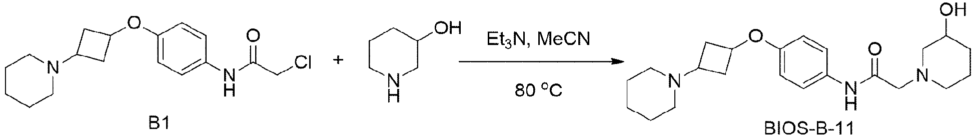

Referred to the synthesis route of compound BIOS-B-1 in Example 1, in this example, 3-hydroxypyridine ethanol from this example was used instead of morpholine from Example 1, and the remaining preparation steps were the same as in Example 1. 108 mg brown oily liquid **(compound BIOS-B-11)** was obtained with a yield of 89.90%.

Furthermore, the hydrochloride of compound BIOS-B-8 could be prepared by reacted with HCl/EA for 1-2 hours, which afford 108 mg white solid with a yield of 84.16%.
MS:388.1[M+H]⁺_{∘}
¹H NMR (400 MHz, DMSO-*d*₆) δ 9.54 (s, 1H), 7.50 (d, *J* = 9.0 Hz, 2H), 6.76 (d, *J* = 9.0 Hz, 2H), 4.82~4.53 (m, 2H), 3.63~3.57 (m, 1H), 3.05 (d, *J =* 1.7 Hz, 2H), 2.93~2.81 (m, 1H), 2.73 (d, *J =* 10.7, 1H), 2.57 (d*, J =* 10.7, 1H), 2.39~2.02 (m, 10H), 1.72~1.69 (m, 2H), 1.57~1.34 (m, 7H), 1.30~1.09 (m, 1H).

### Example 12: Preparation of 2-(4-cyclobutylpiperazin-1-yl)-N-(4-(3-(piperidin-1-yl)cyclobutoxy) phenyl)acetamide (BIOS-B-12)

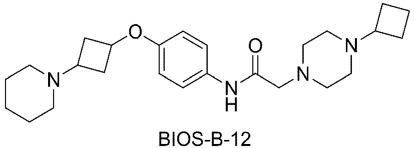

The synthesis route is as follows:

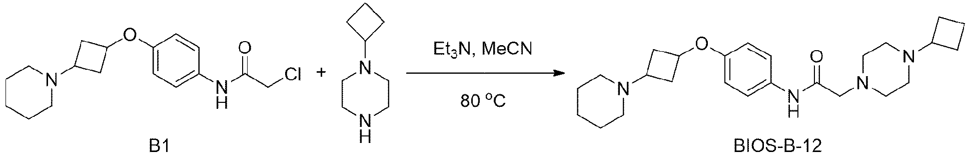

Referred to the synthesis route of compound BIOS-B-3 in Example 3, in this example, 4-cyclobutylpiperazine from this example was used instead of piperidine from Example 3, and the remaining preparation steps were the same as in Example 3. 50 mg pale yellow solid **(compound BIOS-B-12)** was obtained with a yield of 37.81%.
MS: 427.3[M+H]⁺.
¹H NMR (400 MHz, DMSO-*d*₆) δ 9.52 (s, 1H), 7.50 (d, *J =* 9.0 Hz, 2H), 6.75 (d, *J =* 9.0 Hz, 2H), 4.69 (s, 1H), 3.07 (s, 2H), 2.95 (s, 1H), 2.74 (s, 1H), 2.45~2.04 (m, 12H), 2.02~1.87 (m, 2H), 1.78 (t, *J =* 9.0 Hz, 2H), 1.70~1.58 (m, 2H), 1.58~1.31 (m, 7H), 1.29~1.20 (m, 1H), 1.16~0.80 (m, 2H).

### Example 13: Preparation of 1-(2-morpholinoethyl)-3-(4-(3-(piperidin-1-yl)cyclobutoxy)phenyl)urea (BIOS-B-13)

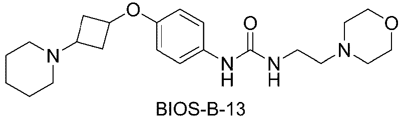

The synthesis route is as follows:

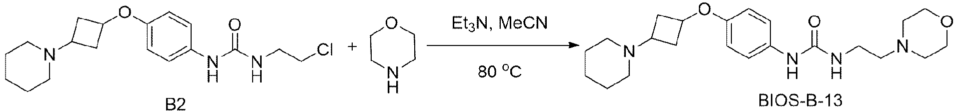

Intermediate B2 (100 mg, 0.284 mmol), morpholine (75 µL, 0.853 mmol), and 200 µL triethylamine were dissolved in 4 mL acetonitrile and transferred to reaction overnight in 80 °C oil bath. The reaction of the raw material was complete as detected by thin layer chromatography.The reaction solution was concentrated to dryness and the crude product was purified on a preparative plate (ethyl acetate: triethylamine =4:1), affording 99 mg white solid **(compound BIOS-B-13),** with a yield of 86.60%.
MS:403.1[M+H]⁺.
¹H NMR (400 MHz, DMSO-*d*₆) δ 8.40 (s, 1H), 7.25 (d, *J =* 9.0 Hz, 2H), 6.68 (d, *J =* 9.0 Hz, 2H), 5.95 (t, *J =* 5.4 Hz, 1H), 4.68~4.59 (m, 1H), 3.59 (t, *J* = 4.7 Hz, 4H), 3.25~3.15 (m, 2H), 2.95~2.83 (m, 1H), 2.42~2.21 (m, 12H), 2.15~2.05 (m, 2H), 1.55~1.32 (m, 6H).

### Example 14: Preparation of (R)-1-(2-(2-methylpyrrolidin-1-yl)ethyl)-3-(4-(3-(piperidin-1-yl)cy clobutoxy)phenyl)urea (BIOS-B-14)

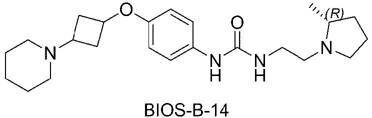

The synthesis route is as follows:

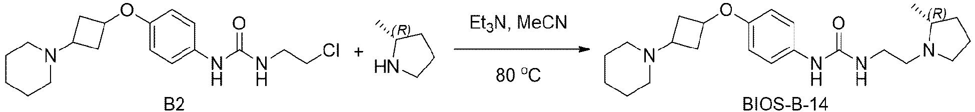

Referred to the synthesis route of compound BIOS-B-13 in Example 13, in this example, (R)-2-methylpyrrolidine from this example was used instead of morpholine from Example 13, and the remaining preparation steps were the same as in Example 13. 45 mg white solid **(compound BIOS-B-14)** was obtained with a yield of 39.56%.
MS:401.2[M+H]⁺.
¹H NMR (400 MHz, DMSO-*d*₆) δ 8.42 (s, 1H), 7.25 (d, *J =* 8.4 Hz, 2H), 6.68 (d, *J =* 8.4 Hz, 2H), 5.94 (s, 1H), 4.63 (s, 1H), 3.09 (s, 2H), 2.84 (s, 2H), 2.40~1.99 (m, 11H), 1.96~1.82 (m, 1H), 1.66 (t, *J =* 7.8 Hz, 2H), 1.58~1.20 (m, 8H), 1.04 (d, *J =* 4 Hz, 3H).

### Example 15: Preparation of 1-(4-(3-(piperidin-1-yl)cyclobutoxy)phenyl)-3-(2-(piperidin-1-yl)et hyl)urea (BIOS-B-15)

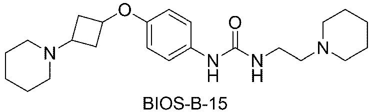

The synthesis route is as follows:

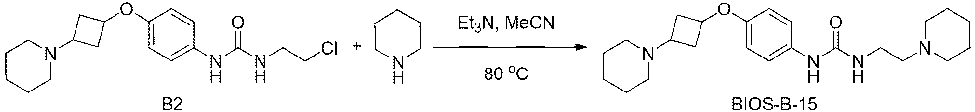

Referred to the synthesis route of compound BIOS-B-13 in Example 13, in this example, piperidine from this example was used instead of morpholine from Example 13, and the remaining preparation steps were the same as in Example 13. 87 mg white solid **(compound BIOS-B-15)** was obtained with a yield of 76.48%.
MS:401.1[M+H]⁺.
¹H NMR (400 MHz, DMSO-*d*₆) δ 8.42 (s, 1H), 7.25 (d, *J =* 9.0 Hz, 2H), 6.68 (d, *J =* 9.0 Hz, 2H), 5.92 (t, *J* = 5.3 Hz, 1H), 4.68~4.58 (m, 1H), 3.17 (q, *J* = 6.0 Hz, 2H), 2.86 (s, 1H), 2.46~2.15 (m, 12H), 2.14~2.03 (m, 2H), 1.58~1.30 (m, 12H).

### Example 16: Preparation of 1-(2-(piperazin-1-yl)ethyl)-3-(4-(3-(piperidin-1-yl)cyclobutoxy)phe nyl)urea (BIOS-B-16)

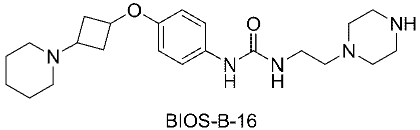

The synthesis route is as follows:

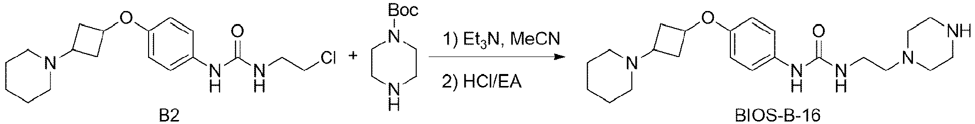

Intermediate B2 (300 mg, 0.852 mmol), 1-Boc-Piperazine (477 mg, 2.558 mmol), and 600 µL triethylamine were dissolved in 4 mL acetonitrile and transferred to reaction overnight in 80 °C oil bath. The reaction of the raw material was complete as detected by thin layer chromatography.

The reaction solution was concentrated to dryness and the crude product was purified on a preparative plate (ethyl acetate: triethylamine = 4: 1), affording 346 mg white solid Intermediate. 207 mg of the intermediate was reacted with HCl/EA for 1-2 hours, affording 219 mg white solid **(the hydrochloride of compound BIOS-B-16),** with a two-step yield of 50.31%.
MS:402.1[M+H]⁺.
¹H NMR (400 MHz, DMSO-*d*₆) δ 11.35 (s, 1H), 8.42 (s, 1H), 7.27 (d, *J* = 9.0 Hz, 2H), 6.69 (d, *J =* 9.0 Hz, 2H), 5.95 (t, *J =* 5.5 Hz, 1H), 4.68~4.59 (m, 1H), 3.22~3.16 (m, 2H), 2.86 (s, 1H), 2.46~2.15 (m, 12H), 2.14~2.03 (m, 2H), 1.58~1.30 (m, 10H).

### Example 17: Preparation of 1-(2-(4-methyl-1,4-diazepan-1-yl)ethyl)-3-(4-(3-(piperidin-1-yl)cycl obutoxy)phenyl)urea (BIOS-B-17)

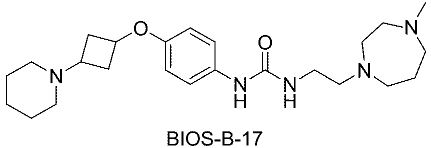

The synthesis route is as follows:

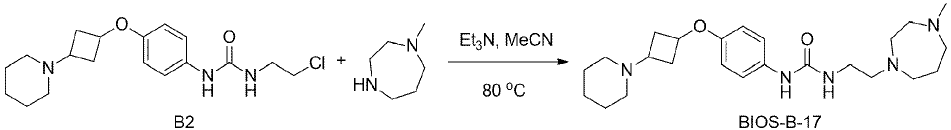

Referred to the synthesis route of compound BIOS-B-13 in Example 13, in this example, 1-methyl-1,4-diazepane from this example was used instead of morpholine from Example 13, and the remaining preparation steps were the same as in Example 13. 74 mg yellow solid **(compound BIOS-B-17)** was obtained with a yield of 60.65%.
MS:430.2[M+H]⁺.
¹H NMR (400 MHz, DMSO-*d*₆) δ 8.88 (s, 1H), 7.29 (d, *J =* 9.0 Hz, 2H), 6.69 (d, *J =* 9.0 Hz, 2H), 6.43 (t, *J =* 5.3 Hz, 1H), 4.70~4.64 (m, 1H), 3.24~3.21 (m, 4H), 3.18~3.13 (m, 5H), 3.05~3.03 (m, 2H), 2.85 (t*, J* = 5.2 Hz, 2H), 2.71 (d, *J =* 3.9 Hz, 5H), 2.62~2.58 (m, 3H), 2.18 (t, *J =* 9.9 Hz, 2H), 1.99~1.89 (m, 2H), 1.64~1.39 (m, 6H), 1.18 (t, *J* = 7.3 Hz, 1H).

### Example 18: Preparation of 1-(2-(4,4-difluoropiperidin-1-yl)ethyl)-3-(4-(3-(piperidin-1-yl)cyclo butoxy)phenyl)urea (BIOS-B-18)

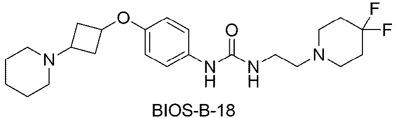

The synthesis route is as follows:

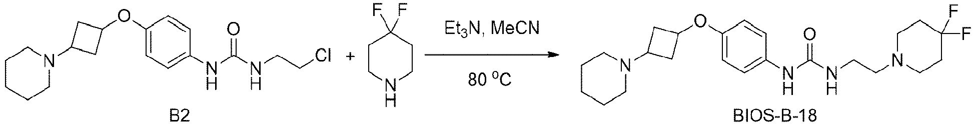

Referred to the synthesis route of compound BIOS-B-13 in Example 13, in this example, 4,4-difluoropiperidine from this example was used instead of morpholine from Example 13, and the remaining preparation steps were the same as in Example 13. 97 mg yellow solid **(compound BIOS-B-18)** was obtained with a yield of 78.24%.
MS:437.1[M+H]⁺.
¹H NMR (400 MHz, DMSO-*d*₆) δ 8.36 (s, 1H), 7.25 (d, *J =* 9.0 Hz, 2H), 6.68 (d, *J =* 9.0 Hz, 2H), 5.96 (t, *J =* 5.5 Hz, 1H), 4.68~4.58 (m, 1H), 3.18 (q, *J =* 6.1 Hz, 2H), 2.87 (s, 1H), 2.58~2.51 (m, 4H), 2.45 (t, *J* = 6.4 Hz, 2H), 2.37~2.16 (m, 6H), 2.14~2.03 (m, 2H), 2.02~1.88 (m, 4H), 1.56~1.33 (m, 6H).

### Example 19: Preparation of 1-(2-(4-hydroxypiperidin-1-yl)ethyl)-3-(4-(3-(piperidin-1-yl)cyclob utoxy)phenyl)urea (BIOS-B-19)

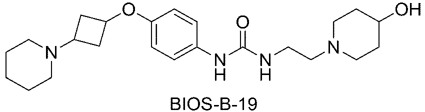

The synthesis route is as follows:

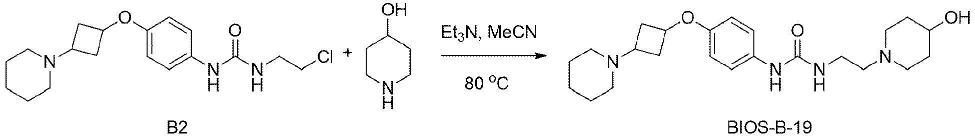

Referred to the synthesis route of compound BIOS-B-13 in Example 13, in this example, 4-hydroxypyridine from this example was used instead of morpholine from Example 13, and the remaining preparation steps were the same as in Example 13. 97 mg pink solid **(compound BIOS-B-19)** was obtained with a yield of 81.99%.
MS:437.1[M+H]⁺.
¹H NMR (400 MHz, DMSO-*d*₆) δ 8.42 (s, 1H), 7.25 (d, *J =* 8.4 Hz, 2H), 6.68 (d, *J =* 8.4 Hz, 2H), 5.93 (t, *J =* 5.4 Hz, 1H), 4.69~4.59 (m, 1H), 4.57 (s, 1H), 3.45 (s, 1H), 3.16 (q, *J =* 6.0 Hz, 2H), 2.93~2.85 (m, 1H), 2.81~2.64 (m, 2H), 2.42~2.16 (m, 8H), 2.14~1.94 (m, 4H), 1.81~1.62 (m, 2H), 1.56~1.29 (m, 8H).

### Example 20: Preparation of 1-(4-(3-(piperidin-1-yl)cyclobutoxy)phenyl)-3-(2-thiomorpholinoet hyl)urea (BIOS-B-20)

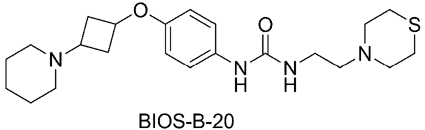

The synthesis route is as follows:

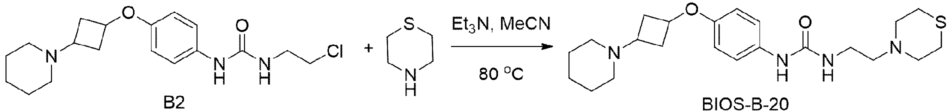

Referred to the synthesis route of compound BIOS-B-13 in Example 13, in this example, thiomorpholine from this example was used instead of morpholine from Example 13, and the remaining preparation steps were the same as in Example 13. 103 mg yellow solid **(compound BIOS-B-20)** was obtained with a yield of 86.64%.
MS:419.1[M+H]⁺.
¹H NMR (400 MHz, DMSO-*d*₆) δ 8.36 (s, 1H), 7.25 (d, *J =* 9.0 Hz, 2H), 6.68 (d, *J =* 9.0 Hz, 1H), 5.91 (t, *J =* 5.4 Hz, 1H), 4.68~4.58 (m, 1H), 3.17 (q, *J =* 6.1 Hz, 2H), 2.92~2.80 (m, 1H), 2.72 ~2.57 (m, 8H), 2.40 (t*, J =* 6.4 Hz, 2H), 2.35~2.16 (m, 6H), 2.14~2.03 (m, 2H), 1.54~1.32 (m, 6H).

### Example 21: Preparation of 1-(2-(2-(2-hydroxyethyl)piperidin-1-yl)ethyl)-3-(4-(3-(piperidin-1-yl)cyclobutoxy)phenyl)urea (BIOS-B-21)

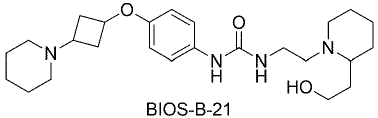

The synthesis route is as follows:

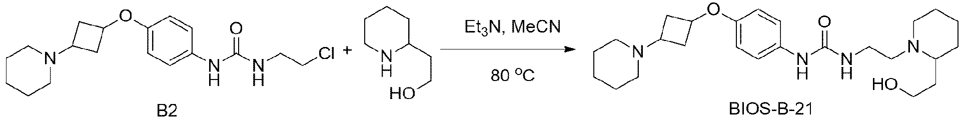

Referred to the synthesis route of compound BIOS-B-13 in Example 13, in this example, 2-piperidine ethanol from this example was used instead of morpholine from Example 13, and the remaining preparation steps were the same as in Example 13. 74 mg yellow solid **(compound BIOS-B-21)** was obtained with a yield of 58.60%.
MS:445.2[M+H]⁺.
¹H NMR (400 MHz, DMSO-*d*₆) δ 8.45 (s, 1H), 7.26 (d, *J =* 8.9 Hz, 2H), 6.68 (d, *J =* 8.9 Hz, 2H), 5.92 (t, *J =* 5.4 Hz, 1H), 4.68~4.58 (m, 1H), 4.32 (s, 1H), 3.25~3.12 (m, 4H), 2.86 (s, 1H), 2.46~2.15 (m, 11H), 2.14~2.03 (m, 2H), 1.58~1.30 (m, 14H).

### Example 22: Preparation of 1-(2-(4-methylpiperazin-1-yl)ethyl)-3-(4-(3-(piperidin-1-yl)cyclobu toxy)phenyl)urea (BIOS-B-22)

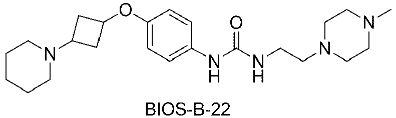

The synthesis route is as follows:

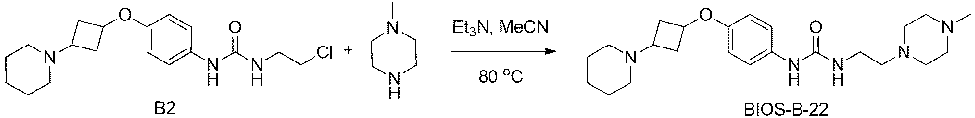

Referred to the synthesis route of compound BIOS-B-13 in Example 13, in this example, 4-methylpiperazine from this example was used instead of morpholine from Example 13, and the remaining preparation steps were the same as in Example 13. 101 mg yellow solid **(compound BIOS-B-22)** was obtained with a yield of 85.58%.
MS:416.2[M+H]⁺.
¹H NMR (400 MHz, DMSO-*d*₆) δ 8.45 (s, 1H), 7.27 (d, *J =* 8.8 Hz, 2H), 6.70 (d, *J =* 8.8 Hz, 2H), 5.97 (s, 1H), 4.68 (s, 1H), 3.22~3.16 (m, 3H), 3.05 (s, 2H), 2.67 (s, 1H), 2.44~2.04 (m, 14H), 1.68~1.32 (m, 7H), 1.29~1.08 (m, 3H).

### Example 23: Preparation of 1-(2-(3-hydroxypiperidin-1-yl)ethyl)-3-(4-(3-(piperidin-1-yl)cyclob utoxy)phenyl)urea (BIOS-B-23)

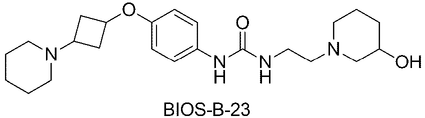

The synthesis route is as follows:

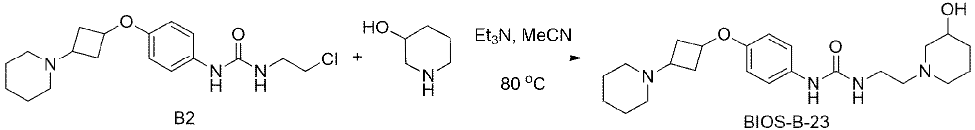

Referred to the synthesis route of compound BIOS-B-13 in Example 13, in this example, 3-hydroxypyridine from this example was used instead of morpholine from Example 13, and the remaining preparation steps were the same as in Example 13. 103 mg yellow solid **(compound BIOS-B-23)** was obtained with a yield of 87.06%.
MS:417.1[M+H]⁺.
¹H NMR (400 MHz, DMSO-*d*₆) δ 8.41 (s, 1H), 7.26 (d, *J =* 9.2 Hz, 2H), 6.68 (d, *J =* 9.2 Hz, 2H), 5.94 (t, *J* = 5.3 Hz, 1H), 4.68~4.61 (m, 1H), 4.61~4.53 (m, 1H), 3.53~3.46 (m, 1H), 3.18~3.12 (m, 2H), 2.91~2.78 (m, 2H), 2.77~2.61 (m, 2H), 2.43~2.17 (m, 8H), 2.14~2.03 (m, 2H), 1.94~1.71 (m, 3H), 1.67~1.57 (m, 1H), 1.54~1.35 (m, 7H).

### Example 24: Preparation of 1-(2-(4-cyclobutylpiperazin-1-yl)ethyl)-3-(4-(3-(piperidin-1-yl)cycl obutoxy)phenyl)urea (BIOS-B-24)

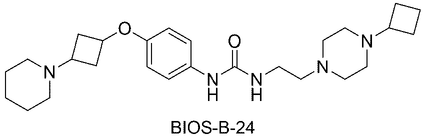

The synthesis route is as follows:

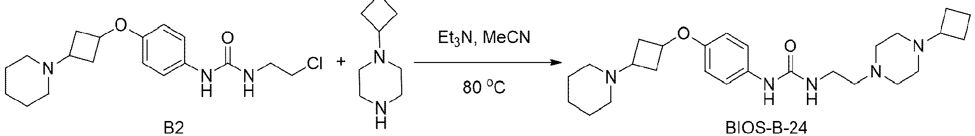

Referred to the synthesis route of compound BIOS-B-13 in Example 13, in this example, 4-cyclobutylpiperazine from this example was used instead of morpholine from Example 13, and the remaining preparation steps were the same as in Example 13. 70 mg yellow solid **(compound BIOS-B-24)** was obtained with a yield of 54.10%.
MS:456.1[M+H]⁺.
¹H NMR (400 MHz, DMSO-*d*₆) δ 8.42 (s, 1H), 7.25 (d, *J =* 9.0 Hz, 2H), 6.67 (d, *J =* 9.0 Hz, 2H), 5.92 (s, 1H), 3.18~3.14 (m, 2H), 2.88~2.80 (m, 1H), 2.67 (t, *J* = 7.7 Hz, 1H), 2.45~2.01 (m, 18H), 1.93 (s, 2H), 1.82~1.56 (m, 5H), 1.55~1.32 (m, 6H).

### Example 25: Preparation of 2-morpholino-N-(4-(3-(piperidin-1-yl)cyclobutoxy)phenyl)ethanet hioamide (BIOS-B-25)

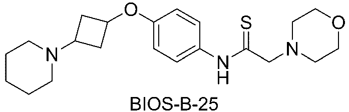

The synthesis route is as follows:

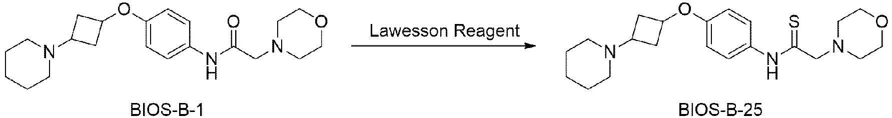

Compound BIOS-B-1 (100 mg, 0.268 mmol) and Lawesson Reagent (108 mg, 0.268 mmol) were dissolved in 4 mL anhydrous 1,4-dioxane and refluxed for 16 hours. The reaction of the raw material was complete as detected by thin layer chromatography.

The reaction solution was concentrated to dryness and the crude product was purified on a preparative plate (petroleum ether: ethyl acetate: triethylamine = 5: 4: 1), affording 12 mg yellow solid **(compound BIOS-B-25),** with a yield of 11.46%.
MS:390.1[M+H]⁺.
¹H NMR (400 MHz, DMSO-*d*₆) δ 9.59 (s, 1H), 7.50 (d, *J* = 9.0 Hz, 2H), 6.75 (d, *J* = 9.0 Hz, 2H), 4.70~4.65 (m, 1H), 3.70~3.54 (m, 4H), 3.14 (s, 2H), 2.88~2.81 (m, 1H), 2.50~2.46 (m, 4H), 2.37~2.00 (m, 8H), 1.60~1.35 (m, 6H).

The synthesis route and preparation steps of compound BIOS-B-1 are described in Example 1 and will not be elaborated here.

### Example 26: Preparation of (R)-2-(2-methylpyrrolidin-1-yl)-N-(4-(3-(piperidin-1-yl)cyclobutox y)phenyl)ethanethioamide (BIOS-B-26)

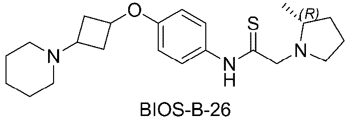

The synthesis route is as follows:

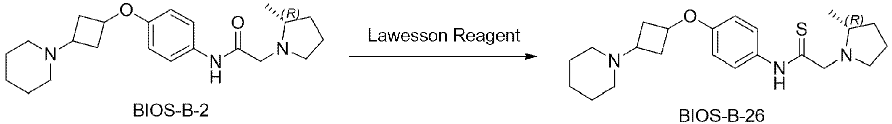

Referred to the synthesis route of compound BIOS-B-25 in Example 25, in this example, BIOS-B-2 from this example was used instead of BIOS-B-1 from Example 25, and the remaining preparation steps were the same as in Example 25. 38 mg yellow solid **(compound BIOS-B-26)** was obtained with a yield of 36.45%.
MS:388.1[M+H]⁺.
¹H NMR (400 MHz, DMSO-*d*₆) δ 9.47 (s, 1H), 7.50 (d, *J* = 9.0 Hz, 2H), 6.75 (d, *J* = 9.0 Hz, 2H), 4.69~4.64 (m, 1H), 3.32 (d, *J =* 15.5 Hz, 1H), 3.11~3.06 (m, 1H), 2.90 (d, *J =* 15.5 Hz, 1H), 2.88~2.81 (m, 1H),2.49-2.03 (m, 10H), 1.97~1.87 (m, 1H), 1.81~1.60 (m, 2H), 1.57~1.29 (m, 7H), 1.06 (d, *J* = 6.1 Hz, 3H).

The synthesis route and preparation steps of compound BIOS-B-2 are described in Example 2 and will not be elaborated here.

### Example 27: Preparation of 2-(piperidin-1-yl)-N-(4-(3-(piperidin-1-yl)cyclobutoxy)phenyl)etha nethioamide (BIOS-B-27)

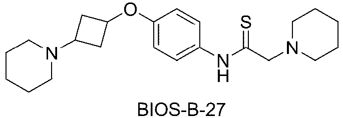

The synthesis route is as follows:

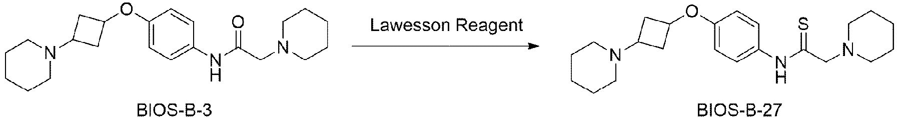

Referred to the synthesis route of compound BIOS-B-25 in Example 25, in this example, BIOS-B-3 from this example was used instead of BIOS-B-1 from Example 25, and the remaining preparation steps were the same as in Example 25. 29 mg yellow solid **(compound BIOS-B-27)** was obtained with a yield of 27.81%.
MS:388.1[M+H]⁺.
¹H NMR (400 MHz, DMSO-*d*₆) δ 9.50 (s, 1H), 7.50 (d, *J* = 9.0 Hz, 2H), 6.75 (d, *J* = 9.0 Hz, 2H), 4.70~4.65 (m, 1H), 3.10 (s, 2H), 2.95~2.81 (m, 1H), 2.44 (t, *J =* 5.3 Hz, 4H), 2.38~2.03 (m, 8H), 1.66~1.31 (m, 12H).

The synthesis route and preparation steps of compound BIOS-B-3 are described in Example 3 and will not be elaborated here.

### Example 28: Preparation of 2-(4-methyl-1,4-diazepan-1-yl)-N-(4-(3-(piperidin-1-yl)cyclobutox y)phenyl)ethanethioamide (BIOS-B-28)

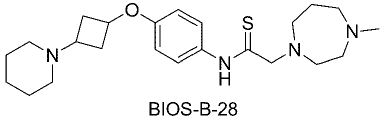

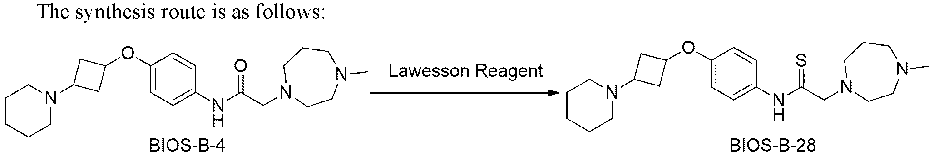

Referred to the synthesis route of compound BIOS-B-25 in Example 25, in this example, BIOS-B-4 from this example was used instead of BIOS-B-1 from Example 25, and the remaining preparation steps were the same as in Example 25. 30 mg yellow solid **(compound BIOS-B-28)** was obtained with a yield of 37.90%.
MS:417.1[M+H]⁺.
¹H NMR (400 MHz, DMSO-*d*₆) δ 9.53 (s, 1H), 7.50 (d, *J* = 9.0 Hz, 2H), 6.75 (d, *J* = 9.0 Hz, 2H), 4.70~4.64 (m, 1H), 3.28 (s, 2H), 2.87~2.83 (m, 1H), 2.80~2.71 (m, 4H), 2.64~2.56 (m, 4H), 2.38~2.14 (m, 9H), 2.12~2.06 (m, 2H), 1.83~1.71 (m, 2H), 1.56~1.32 (m, 6H).

The synthesis route and preparation steps of compound BIOS-B-4 are described in Example 4 and will not be elaborated here.

### Example 29: Preparation of 2-(diethylamino)-N-(4-(3-(piperidin-1-yl)cyclobutoxy)phenyl)aceta mide (BIOS-B-29)

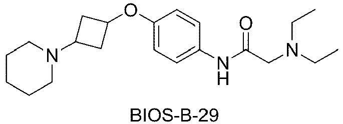

The synthesis route is as follows:

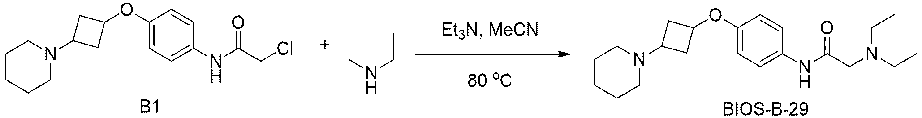

Referred to the synthesis route of compound BIOS-B-1 in Example 1, in this example, diethylamine from this example was used instead of morpholine from Example 1, and the remaining preparation steps were the same as in Example 1. 69 mg pale yellow **(compound BIOS-B-29)** was obtained with a yield of 61.95%.
MS:360.1[M+H]⁺.
¹H NMR (400 MHz, DMSO-*d*₆) δ 9.49 (s, 1H), 7.50 (d, *J* = 8.8 Hz, 2H), 6.75 (d, *J* = 8.8 Hz, 2H), 4.70~4.65 (m, 1H), 3.18 (s, 2H), 2.95~2.81 (m, 1H), 2.44 (t, *J =* 5.5 Hz, 4H), 2.29~2.08 (m, 8H), 1.66~1.26 (m, 12H).

### Example 30: Synthesis of 2-(dimethylamino)-N-(4-(3-(piperidin-1-yl)cyclobutoxy)phenyl)aceta mide (BIOS-B-30)

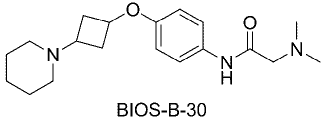

The synthesis route is as follows:

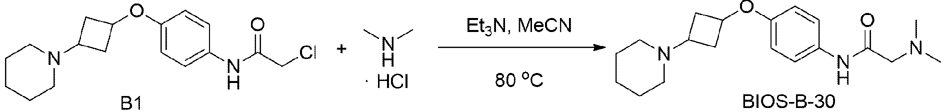

Referred to the synthesis route of compound BIOS-B-1 in Example 1, dimethylamine hydrochloride from this example was used instead of morpholine from Example 1, and the remaining preparation steps were the same as in Example 1. 51 mg pale yellow **(compound BIOS-B-30)** was obtained with a yield of 49.67%.
MS:332.1[M+H]⁺.
¹H NMR (400 MHz, DMSO-*d*₆) δ 9.56 (s, 1H), 7.51 (d, *J* = 8.9 Hz, 2H), 6.77 (d, *J* = 8.9 Hz, 2H), 4.70~4.65 (m, 1H), 3.12 (s, 2H), 2.95~2.82 (m, 1H), 2.45 (t, *J =* 5.6 Hz, 4H), 2.29~2.08 (m, 10H), 1.66~1.26 (m, 6H).

### Example 31: Preparation of 2-(2-(dimethylamino)ethoxy)-N-(4-(3-(piperidin-1-yl)cyclobutoxy) phenyl)acetamide (BIOS-B-31)

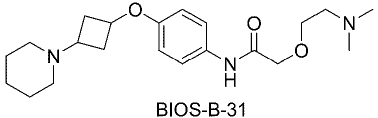

The synthesis route is as follows:

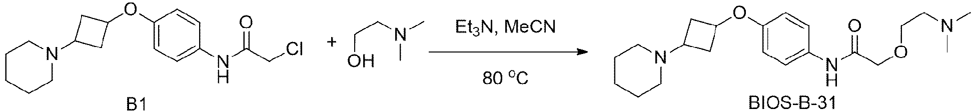

Referred to the synthesis route of compound BIOS-B-1 in Example 1, N,N-Dimethylethanolamine from this example was used instead of morpholine from Example 1, and the remaining preparation steps were the same as in Example 1.67 mg pale yellow **(compound BIOS-B-31)** was obtained with a yield of 57.58%.
MS:376.1[M+H]⁺.
¹H NMR (400 MHz, DMSO-*d*₆) δ 9.26 (s, 1H), 7.53 (d, *J =* 8.6 Hz, 2H), 6.85 (d, *J =* 8.6 Hz, 2H), 4.65 (t, *J* = 9.9 Hz, 1H), 4.16 (s, 2H), 3.69 (t, *J* = 6.7 Hz, 2H), 3.02 (t, *J* = 6.3 Hz, 1H), 2.81~2.69 (m, 5H), 2.58~2.43 (m, 3H), 2.36~2.30 (m, 3H), 2.18~2.02 (m, 3H), 1.57~1.38 (m, 8H).

### Example 32: The antagonistic of compounds target to Histamine H3 receptor

The FLPPR assay (NIH Assay Guidance Manual: HTS Assay Validation-Section 4.3. Analysis (Potency), htpps://www.ncbi.nlm.nih.gov/books/NBK83783) was performed using SUVN-G3031 as the positive control, and 21 target compounds were tested for the antagonistic activity target to histamine H3 receptor. The results are shown in Table 1.

**Table 1. The antagonistic of compounds target to Histamine H3 receptor**

| **Compound ID** | **%Inh-AVERAGE @ 50 nM** | **%Inh-AVERAGE @ 5 nM** |
|---|---|---|
| Positive control SUVN-G3031 | 85.82 | 57.76 |
| BIOS-B-1 | 100.18 | 91.74 |
| BIOS-B-2 | 100.21 | 100.12 |
| BIOS-B-3 | 99.37 | 95.56 |
| BIOS-B-4 | 100.21 | 101.36 |
| BIOS-B-5 | 99.89 | 98.88 |
| BIOS-B-6 | 99.75 | 102.75 |
| BIOS-B-7 | 99.51 | 88.27 |
| BIOS-B-8 | 99.46 | 101.42 |
| BIOS-B-9 | 99.60 | 100.64 |
| BIOS-B-10 | 97.46 | 99.46 |
| BIOS-B-11 | 99.43 | 97.00 |
| BIOS-B-12 | 98.81 | 99.08 |
| BIOS-B-13 | 99.76 | 99.56 |
| BIOS-B-14 | 100.58 | 100.07 |
| BIOS-B-15 | 99.95 | 98.97 |
| BIOS-B-17 | 99.40 | 100.54 |
| BIOS-B-18 | 99.87 | 99.72 |
| BIOS-B-19 | 100.86 | 100.62 |
| BIOS-B-20 | 100.51 | 100.27 |
| BIOS-B-22 | 99.52 | 101.10 |
| BIOS-B-23 | 100.31 | 100.47 |

As shown in Table 1, some compounds showed strong histamine H3 receptor antagonist activity, and most of them are more active than the positive control at 5 nM.

### Example 33: The hERG inhibition of compounds

In cardiomyocytes, inhibition of the delayed rectifier potassium current (IKr) channel protein encoded by the human Ether-a-go-go Related Gene (hERG) is the most important mechanism by which drugs lead to prolongation of the QT interval. hERG is characterized by a specific molecular structure, which allows it to be inhibited by different structures, leading to severe arrhythmias. Statistically, 25-40% of lead compounds show varying degrees of hERG-related toxicity. Terefore, early evaluation of the effects of compounds on hERG is crucial in the drug development process.

The inhibition rate of compounds on hERG ion channels was examined by manual membrane clamp technique on HEK293 cell line which is stably expresses hERG ion channels. 100 nM of hERG inhibitor Cisapride was used as positive control.

The results are shown in Table 2. The inhibition rate of compounds, such as BIOS-B-17∼BIOS-B-23, BIOS-B-8∼BIOS-B-15, BIOS-B-6, and BIOS-B-4 (10 µM), showed less than 5% on hERG channel, which indicated that the tested compounds have a good security.

**Table 2. The inhibition rate of compounds on hERG channel**

| Compound ID | Average inhibition rate (%) | |
|---|---|---|
| | 10 µM | 100 nM |
| Cisapride | - | 86.6 |
| BIOS-B-1 | 8.54 | - |
| BIOS-B-4 | 0.46 | - |
| BIOS-B-6 | -0.49 | - |
| BIOS-B-8 | 4.82 | - |
| BIOS-B-9 | -1.03 | - |
| BIOS-B-10 | -1.26 | - |
| BIOS-B-11 | 2.96 | - |
| BIOS-B-12 | 2.06 | - |
| BIOS-B-13 | -1.47 | - |
| BIOS-B-14 | 0.17 | - |
| BIOS-B-15 | -0.12 | - |
| BIOS-B-17 | 1.52 | - |
| BIOS-B-18 | -0.65 | - |
| BIOS-B-19 | -0.09 | - |
| BIOS-B-20 | -0.66 | - |
| BIOS-B-22 | -2.43 | - |
| BIOS-B-23 | 0.37 | - |

| | | |
|---|---|---|
| Note:"-" refers not measured. | | |

### Example 34: The analgesic effects of compounds BIOS-B-12 and BIOS-B-14

By exposing the sciatic nerve on male SD rats' (180-200 g) right hind leg, in the anterior segment where the sciatic nerve is about to bifurcate, sterile chromic gut thread (No. 4, 0.15 mm in diameter) was used to ligate 4 loops loosely, with each loop 1-2 mm apart, and the muscle and skin were sutured to establish the chronic constriction injury (CCI) model. In the sham-operated group, only the sciatic nerve was exposed without ligation, and the muscle and skin were sutured. The mechanical withdrawal threshold was detected by electronic analgesia meter (IITC-2391) on the 7^{th} day for 2 consecutive days.

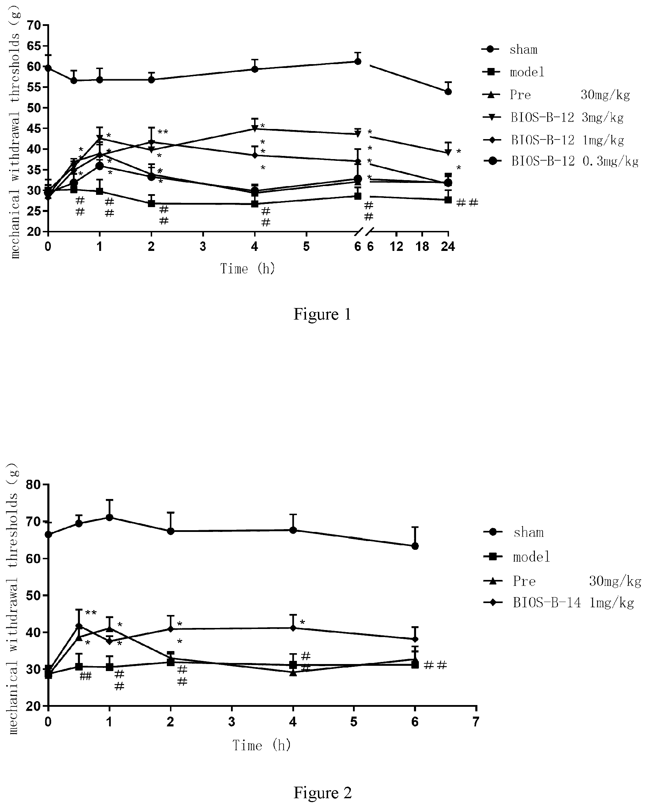

The CCI model rats with stabilized mechanical withdrawal thresholds were randomly divided into five groups, named model group (model), pregabalin group (Pre, 30 mg/kg), BIOS-B-12 compound group (3 mg/kg), BIOS-B-12 compound group (1 mg/kg), and BIOS-B-12 compound group (0.3 mg/kg), and were administered at 1 mL/100 g by gavage, and the same volume of saline was gavaged in Sham an model group. The basic value was determined before drug administration, and the mechanical withdrawal thresholds were detected at 0.5h, 1h, 2h, 4h, 6h, and 24h after a single drug administration, and the p value was calculated by ANOVA with T-test. The results are shown in Figure 1.

The results showed that after the injection of BIOS-B-12 at three doses of 3, 1, and 0.3 mg/kg in a single dose, respectively, a dose-dependently increasing of the mechanical withdrawal thresholds in the CCI model rats was obsevered. BIOS-B-12 (3 mg/kg) at the time point of 0.5h to 24h after the administration of the compound were significantly different from those of the model group (P < 0.01). BIOS-B-12 (1 mg/kg) at the time point of 0.5h to 6h after the administration of the compound were significantly different from those of the model group (P < 0.01). BIOS-B-12 (0.3 mg/kg) at the time point of 1h to 2h after the administration of the compound were significantly different from those of the model group (P < 0.05). Pregabalin (30 mg/kg) only increased the mechanical withdrawl thresholds of CCI rats from 0.5h to 2h after administration, which was significantly different from that of the model group, indicating that the compound BIOS-B-12 acted for a longer period of analgesia time, and the level of analgesia was superior to that of pregabalin.

The CCI model rats with stabilized mechanical withdrawal thresholds were randomly divided into three groups, named model group (model), pregabalin group (Pre, 30 mg/kg), and BIOS-B-14 compound group (1 mg/kg), and were administered at 1 mL/100 g by gavage, and the same volum of saline was gavaged in Sham and model group. The basic value was determined before drug administration, and the mechanical withdrawal thresholds were detected at 0.5h, 1h, 2h, 4h, and 6h after a single drug administration, and the p value was calculated by ANOVA with T-test. The results are shown in Figure 2.

The results showed that for the CCI neuropathic pain model in SD rats, the compound BIOS-B-14 (1 mg/kg) significantly increased the mechanical withdrawal thresholds, and the mechanical withdrawal thresholds at the time points from 0.5h to 5h after administration of the compound were significantly different from those of the model group (P < 0.05). Pregabalin (30 mg/kg) only increased the mechanical withdrawl thresholds of CCI rats from 0.5h to 1h after administration, which was significantly different from that of the model group, indicating that the compound BIOS-B-14 acted for a longer period of analgesia time than that of pregabalin.

### Example 35: Antipruritic ability of compounds BIOS-B-12 and BIOS-B-18

The C57BL/6J male mice, with 6-8 weeks old, were randomly divided into model group (Model), BIOS-B-12 compound group (10 mg/kg), BIOS-B-18 compound group (10 mg/kg) and dexamethasone group (Dex, 3 mg/kg), with 7 animals in each group. The mice were injected subcutaneously with 500 µg/50 µL histamine at the back of the neck after the administration of dexamethasone for 1h, BIOS-B-12 for 4h, and BIOS-B-18 for 1h, respectively, and immediately observed for 30 min. The number of scratched was recorded and the inhibition rate was calculated as (Model-Compound)/Model × 100%.

**Table 3. Antipruritic ability of compounds BIOS-B-12 and BIOS-B-18**

| Compound ID | Inhibition Rate (%) |
|---|---|
| BIOS-B-12 | 36.6 |
| BIOS-B-18 | 55.0 |
| Dex | 29.3 |

As shown in Table 3, BIOS-B-12 (10 mg/kg) and BIOS-B-18 (10 mg/kg) reduced the number of histamine-induced scratched in mice and were more effective than the positive control decamethasone.

## Claims

1. A histamine H3 receptor antagonist, which is a compound of general formula I or a pharmaceutically acceptable salt thereof: 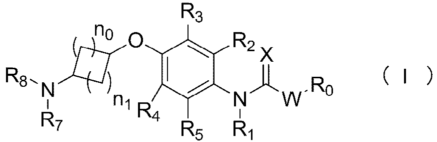 wherein:
W is selected from -(CH₂)ₘ or -NR₆(CH₂)ₘ-;
X is selected from O or S;
n₀ and n₁ are each independently selected from 1, 2 or 3; m is selected from 0, 1, 2, 3, 4, 5 or 6;
R₀ is selected from -NR'R", -O(CH₂)ₘNR'R", substituted or unsubstituted 5-8 membered heterocyclyl, and substituted or unsubstituted 5-8 membered heteroaryl; wherein the heterocyclyl or heteroaryl is optionally each independently substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₁ and R₆ are each independently selected from hydrogen, C₁₋₆ alkyl, -C(O)-alkyl or -S(O)₂-alkyl;
R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
or, R₂ and R₃ together with the carbon atoms on the benzene ring to which they are attached form a substituted or unsubstituted benzobicyclic structure, wherein the benzobicyclic structure can be but not limited to, a benzoheterocyclic ring;
or, R₄ and R₅ together with the carbon atoms on the benzene ring to which they are attached form a substituted or unsubstituted benzobicyclic structure, wherein the benzobicyclic structure can be but not limited to, a benzoheterocyclic ring;
R₇ and R₈ are each independently selected from one or more of hydrogen, halogen, C₁₋₆ alkyl, C₂₋₆ alkenyl, C₂₋₆ alkynyl, amino, cyano, amide, sulfone, sulfoxide, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, C₁₋₆ alkoxyamide, C₁₋₆ alkoxysulfone and C₁₋₆ alkoxysulfoxide;
or, R₇ and R₈ together with the nitrogen atom to which they are attached form a ring, and the substituents on the ring are selected from one or more of the following groups: hydrogen, halogen, C₁₋₆ alkyl, C₁₋₆ alkoxy, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-8 membered heterocyclyl, and 3-8 membered heteroaryl;
the heterocyclyl, heteroaryl and benzoheterocyclic group contain at least one heteroatom, and the heteroatom is selected from N, O or S.

2. The histamine H3 receptor antagonist according to claim 1, which is a compound of general formula II or a pharmaceutically acceptable salt thereof: 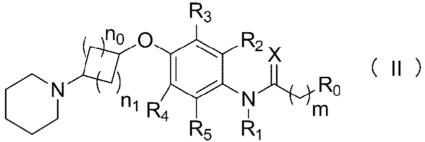 wherein:
X is selected from O or S;
n₀ and n₁ are each independently selected from 1, 2 or 3; m is selected from 0, 1, 2, 3, 4, 5 or 6;
R₀ is selected from -NR'R", -O(CH₂)ₘNR'R", substituted or unsubstituted 5-8 membered heterocyclyl, and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆ alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₁ is selected from hydrogen, C₁₋₆ alkyl, -C(O)-alkyl or -S(O)₂-alkyl;
R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
or, R₂ and R₃ together with the carbon atoms on the benzene ring to which they are attached form a substituted or unsubstituted benzobicyclic structure, wherein the benzobicyclic structure can be but not limited to, a benzoheterocyclic ring;
or, R₄ and R₅ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

3. The histamine H3 receptor antagonist according to claim 2, which is a c compound of general formula II-1 or a pharmaceutically acceptable salt thereof: 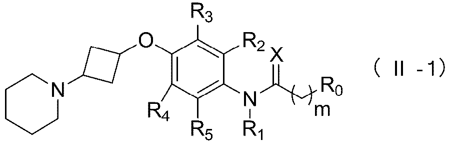 wherein:
X is selected from O or S;
m is selected from 1 or 2;
R₀ is selected from substituted or unsubstituted 5-8 membered heterocyclyl and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R₁ is selected from hydrogen, C₁₋₆ alkyl, -C(O)-alkyl or -S(O)₂-alkyl;
R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
the heterocyclyl and heteroaryl contain at least one heteroatom, and the heteroatom is selected from N, O or S.

4. The histamine H3 receptor antagonist according to claim 2, which is a compound of general formula II-2 or a pharmaceutically acceptable salt thereof: 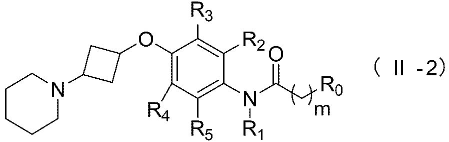 wherein:
m is selected from 1 or 2;
R₀ is selected from -NR'R" or -O(CH₂)mNR'R";
R' and R" are each independently selected from one or more of hydrogen, C₁₋₆ alkyl, C₁₋₆ alkoxy, halogen, amino, hydroxy, carboxyl, carbonyl, amide, cyano, C₁₋₆ haloalkyl, C₁₋₆alkylhydroxy, C₁₋₆ alkylamino, C₁₋₆ alkylamide, C₁₋₆ haloalkoxy, C₁₋₆ alkoxyhydroxy, C₁₋₆ alkoxyamino, and C₁₋₆ alkoxyamide, and R' and R" are not hydrogen at the same time;
R₁ is selected from hydrogen, C₁₋₆ alkyl, -C(O)-alkyl or -S(O)₂-alkyl;
R₂, R₃, R₄, and R₅ are each independently selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide.

5. The histamine H3 receptor antagonist according to claim 1, which is a compound of general formula III or a pharmaceutically acceptable salt thereof: 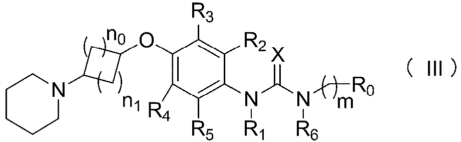 wherein:
X is selected from Oor S;
n₀ and n₁ are each independently selected from 1, 2 or 3; m is selected from 0, 1, 2, 3, 4, 5 or 6;
R₀ is selected from substituted or unsubstituted 5-8 membered heterocyclyl and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, cyano, amide, sulfone, sulfoxide, C₁₋₆ haloalkyl, C₁₋₆ alkylhydroxyl, C₁₋₆ alkylamino, C₁₋₆ alkylamide, C₁₋₆ alkylsulfone, C₁₋₆ alkylsulfoxide, C₁₋₆ haloalkoxy, C₁₋₆ alkoxyhydroxyl, C₁₋₆ alkoxyamino, C₁₋₆ alkoxyamide, C₁₋₆ alkoxysulfone, C₁₋₆ alkoxysulfoxide, 3-6 membered cycloalkyl, 3-6 membered heterocyclyl, aryl and heteroaryl;
R₁ and R₆ are each independently selected from hydrogen, C₁₋₆ alkyl, -C(O)-alkyl or -S(O)₂-alkyl;
R₂, R₃, R₄, R₅ and R₆ are each independently one or more selected from one or more of hydrogen, halogen, C₁₋₆ alkyl, C₂₋₆ alkenyl, C₂₋₆ alkynyl, amino, hydroxyl, cyano, amide, sulfone, sulfoxide, C₁₋₆ haloalkyl, C₁₋₆ alkylhydroxyl, C₁₋₆alkylamino, C₁₋₆ alkylamide, C₁₋₆ alkylsulfone, and C₁₋₆ alkylsulfoxide;
or, R₂ and R₃ together with the carbon atoms on the benzene ring to which they are attached form a substituted or unsubstituted benzobicyclic structure, wherein the benzobicyclic structure can be but not limited to, a benzoheterocyclic ring;
or, R₄ and R₅ together with the carbon atoms on the benzene ring to which they are attached form a substituted or unsubstituted benzobicyclic structure, wherein the benzobicyclic structure can be but not limited to, a benzoheterocyclic ring;
the heterocyclyl, heteroaryl and benzoheterocyclic group contain at least one heteroatom, and the heteroatom is selected from N, O or S.

6. The histamine H3 receptor antagonist according to claim 5, which is a compound of general formula III-1 or a pharmaceutically acceptable salt thereof: 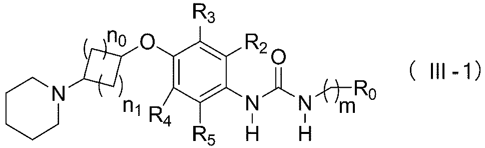 wherein:
n₀ and n₁ are each independently selected from 1, 2 or 3; m is selected from 0, 1, 2, 3, 4, 5 or 6;
R₀ is selected from substituted or unsubstituted 5-8 membered heterocyclyl and substituted or unsubstituted 5-8 membered heteroaryl; wherein the heterocyclyl or heteroaryl is each independently optionally substituted with one or more of C₁₋₆ alkyl, C₁₋₆ alkoxy, C₂₋₆ alkenyl, C₂₋₆ alkynyl, halogen, amino, hydroxyl, C₁₋₆ haloalkyl, C₁₋₆ alkylhydroxyl, C₁₋₆ alkylamino, C₁₋₆ haloalkoxy, C₁₋₆ alkoxyhydroxyl, C₁₋₆ alkoxyamino, 3-6 membered cycloalkyl;
R₂, R₃, R₄, R₅, and R₆ are each independently selected from one or more of hydrogen, halogen, C₁₋₆ alkyl, amino, hydroxy, C₁₋₆ haloalkyl, C₁₋₆ alkylhydroxy, and C₁₋₆ alkylamino;
the heterocyclyl and heteroaryl contain at least one heteroatom, and the heteroatom is selected from N, O or S.

7. A histamine H3 receptor antagonist, which is a compound of the following structure, or a isomer thereof, or a solvate thereof, or a pharmaceutically acceptable salt thereof:
2-morpholino-*N*-(4-(3-(piperidin-1-yl)cyclobutoxy)phenyl)acetamide;
2-(2-methylpyrrolidin-1-yl)-*N*-(4-(3-(piperidin-1-yl)cyclobutoxy)phenyl)acetamide;
2-(piperidin-1-yl)-*N*-(4-(3-(piperidin-1-yl)cyclobutoxy)phenyl)acetamide;
2-(4-methyl-1,4-diazepan-1-yl)-*N*-(4-(3-(piperidin-1-yl)cyclobutoxy)phenyl)acetamide;
2-(4,4-difluoropiperidin-1-yl)-*N*-(4-(3-(piperidin-1-yl)cyclobutoay)phenyl)acetamide;
2-(4-hydroxypiperidin-1-yl)-*N*-(4-(3-(piperidin-1-yl)cyclobutoxy)phenyl)acetamide;
*N*-(4-(3-(piperidin-1-yl)cyclobutoxy)phenyl)-2-thiomorpholinoacetamide;
2-(3-(2-hydroxyethyl)piperidin-1-yl)-*N*-(4-(3-(piperidin-1-yl)cyclobutoxy)phenyl)acetamide;
2-(piperazin-1-yl)-*N*-(4-(3-(piperidin-1-yl)cyclobutoxy)phenyl)acetamide;
2-(4-methylpiperazin-1-yl)-*N*-(4-(3-(piperidin-1-yl)cyclobutoxy)phenyl)acetamide;
2-(3-hydroxypiperidin-1-yl)-*N*-(4-(3-(piperidin-1-yl)cyclobutoxy)phenyl)acetamide;
2-(4-cyclobutylpiperazin-1-yl)-*N*-(4-(3-(piperidin-1-yl)cyclobutoxy)phenyl)acetamide;
1-(2-morpholinoethyl)-3-(4-(3-(piperidin-1-yl)cyclobutoxy)phenyl)urea;
1-(2-(2-methylpyrrolidin-1-yl)ethyl)-3-(4-(3-(piperidin-1-yl)cyclobutoxy)phenyl)urea;
1-(4-(3-(piperidin-1-yl)cyclobutoxy)phenyl)-3-(2-(piperidin-1-yl)ethyl)urea;
1-(2-(piperazin-1-yl)ethyl)-3-(4-(3-(piperidin-1-yl)cyclobutoxy)phenyl)urea;
1-(2-(4-methyl-1,4-diazepan-1-yl)ethyl)-3-(4-(3-(piperidin-1-yl)cyclobutoxy)phenyl)urea;
1-(2-(4,4-difluoropiperidin-1-yl)ethyl)-3-(4-(3-(piperidin-1-yl)cyclobutoxy)phenyl)urea;
1-(2-(4-hydroxypiperidin-1-yl)ethyl)-3-(4-(3-(piperidin-1-yl)cyclobutoxy)phenyl)urea;
1-(4-(3-(piperidin-1-yl)cyclobutoxy)phenyl)-3-(2-thiomorpholinoethyl)urea;
1-(2-(2-(2-hydroxyethyl)piperidin-1-yl)ethyl)-3-(4-(3-(piperidin-1-yl)cyclobutoxy)phenyl)urea;
1-(2-(4-methylpiperazin-1-yl)ethyl)-3-(4-(3-(piperidin-1-yl)cyclobutoxy)phenyl)urea;
1-(2-(3-hydroxypiperidin-1-yl)ethyl)-3-(4-(3-(piperidin-1-yl)cyclobutoxy)phenyl)urea;
1-(2-(4-cyclobutylpiperazin-1-yl)ethyl)-3-(4-(3-(piperidin-1-yl)cyclobutoxy)phenyl)urea;
2-morpholino-*N*-(4-(3-(piperidin-1-yl)cyclobutoxy)phenyl)ethanethioamide;
2-(2-methylpyrrolidin-1-yl)-*N*-(4-(3-(piperidin-1-yl)cyclobutoxy)phenyl)ethancthioamide;
2-(piperidin-1-yl)-*N*-(4-(3-(piperidin-1-yl)cyclobutoxy)phenyl)ethanethioamide;
2-(4-methyl-1,4-diazepan-1-yl)-*N*-(4-(3-(piperidin-1-yl)cyclobutoxy)phenyl)ethanethioamide;
2-(diethylamino)-*N*-(4-(3-(piperidin-1-yl)cyclobutoxy)phenyl)acetamide;
2-(dimethylamino)-*N*-(4-(3-(piperidin-1-yl)cyclobutoxy)phenyl)acetamide; or
2-(2-(dimethylamino)ethoxy)-*N*-(4-(3-(piperidin-1-yl)cyclobutoxy)phenyl)acetamide.

8. A histamine H3 receptor antagonist, which is a compound as the following structure or a pharmaceutically acceptable salt thereof:
(*R*)-2-(2-methylpyrrolidin-1-yl)-*N*-(4-(3-(piperidin-1-yl)cyclobutoxy)phenyl)acetamide;
(*R*)-1-(2-(2-methylpyrrolidin-1-yl)ethyl)-3-(4-(3-(piperidin-1-yl)cyclobutoay)phenyl)urea; or
(*R*)-2-(2-methylpyrrolidin-1-yl)-*N*-(4-(3-(piperidin-1-yl)cyclobutoxy)phenyl)ethanethioamide.

9. A pharmaceutical composition, comprising at least one compound of any one of claims 1-8 or a pharmaceutically acceptable salt thereof, and at least one pharmaceutically acceptable carrier or excipient.

10. Use of a compound of any one of claims 1-8 or the pharmaceutical composition of claim 9 in the manufacture of a medicament for preventing or treating a disease associated with histamine H3 receptor.

11. The use according to claim 10, wherein said the medicament is used to prevent or treat cognitive impairment, dementia, attention deficit hyperactivity disorder, schizophrenia, epilepsy, sleep disorders, sleep apnea, obesity, eating disorders, pain or itching.

12. The use according to claim 10, wherein said the medicament is used to prevent or treat neuropathic pain, which can be but not limited to peripheral neuropathic pain or central neuropathic pain.
